(19) Europäisches Patentamt
European Patent Office
Office européen des brevets

(11) Veröffentlichungsnummer: **0 297 383 B1**

# (12) EUROPÄISCHE PATENTSCHRIFT

(45) Veröffentlichungstag der Patentschrift: **10.10.90**

(51) Int. Cl.[5]: **C07D 249/08**, C07D 233/60, A01N 43/653, A01N 43/50, C07C 49/577

(21) Anmeldenummer: **88109804.0**

(22) Anmeldetag: **20.06.88**

## (54) Hydroxyethyl-azolyl-Derivate.

(30) Priorität: **01.07.87 DE 3721696**
**20.04.88 DE 3813129**

(43) Veröffentlichungstag der Anmeldung:
**04.01.89 Patentblatt 89/1**

(45) Bekanntmachung des Hinweises auf die Patenterteilung:
**10.10.90 Patentblatt 90/41**

(84) Benannte Vertragsstaaten:
**AT BE CH DE ES FR GB GR IT LI NL SE**

(56) Entgegenhaltungen:
**EP-A- 0 040 350**
**EP-A- 0 087 148**
**EP-A- 0 180 136**
**EP-A- 0 196 038**
**EP-A- 0 237 917**

**Die Akte enthält technische Angaben, die nach dem Eingang der Anmeldung eingereicht wurden und die nicht in dieser Patentschrift enthalten sind.**

(73) Patentinhaber: **BAYER AG,**
**D-5090 Leverkusen 1 Bayerwerk(DE)**

(72) Erfinder: **Stroech, Klaus, Dr., Rolsberger Strasse 22,**
**D-5650 Solingen 19(DE)**
Erfinder: **Böckmann, Klaus, Dr., Claudiusweg 7,**
**D-5600 Wuppertal 1(DE)**
Erfinder: **Dutzman, Stefan, Dr., Leinenweberweg 33,**
**D-4000 Düsseldorf 13(DE)**
Erfinder: **Brandes, Wilhelm, Dr., Eichendorffstrasse 3,**
**D-5653 Leichlingen(DE)**
Erfinder: **Lürssen, Klaus, Dr.,**
**August-Kierspel-Strasse 89,**
**D-5060 Bergisch-Gladbach 2(DE)**
Erfinder: **Reinecke, Paul, Dr., Steinstrasse 8,**
**D-5090 Leverkusen 3(DE)**

EP 0 297 383 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

Die vorliegende Erfindung betrifft neue Hydroxyethylazolyl-Derivate, mehrere Verfahren zu deren Herstellung und deren Verwendung als Fungizide und Pflanzenwachstumsregulatoren.

Es ist bereits bekannt, dass zahlreiche Hydroxyalkylazolyl-Derivate fungizide Eigenschaften besitzen (vgl. EP-A 0 040 345 und EP-A 0 061 835). So lässt sich z.B. 1-(4-Chlorphenoxy)-2-cyclopropyl-1-(1,2,4-triazol-1-yl)propan-2-ol zur Bekämpfung von Pilzen einsetzen. Die Wirksamkeit dieses Stoffes ist sehr gut; allerdings lässt die Pflanzenverträglichkeit und die Wirksamkeit in manchen Fällen zu wünschen übrig.

Es wurden nun neue Hydroxyethyl-azolyl-Derivate der Formel

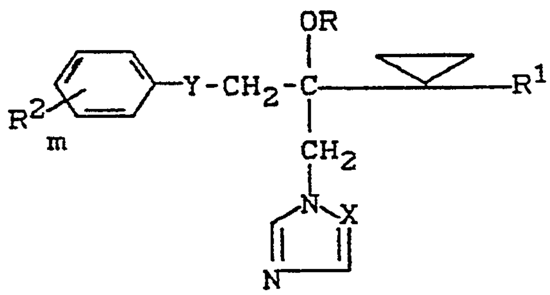

(I)

in welcher
R für Wasserstoff, Alkyl mit 1 bis 6 Kohlenstoffatomen oder Alkylcarbonyl mit 1 bis 6 Kohlenstoffatomen in der Alkylgruppe steht,
$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung $-Z-R^3$ steht, worin
Z für Sauerstoff, Schwefel, SO oder $SO_2$ steht und
$R^3$ für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht,
$R^2$ für Fluor, Chlor, Brom, Methyl, Ethyl, Isopropyl, tert.-Butyl, Methoxy, Methylthio, Trifluormethyl, Trifluormethoxy, Trifluormethylthio, Methoximinomethyl, gegebenenfalls durch Fluor, Chlor und/oder Methyl substituiertes Phenyl oder für gegebenenfalls durch Fluor, Chlor und/oder Methyl substituiertes Phenoxy steht,
m für die Zahlen 0, 1, 2 oder 3 steht,
X für Stickstoff oder eine CH-Gruppe steht und
Y für Sauerstoff, Schwefel, SO oder $SO_2$ steht,
sowie deren Säureadditions-Salze und Metallsalz-Komplexe gefunden.

Weiterhin wurde gefunden, dass man Hydroxyethyl-azolyl-Derivate der Formel (I) sowie deren Säureadditions-Salze und Metallsalzkomplexe erhält, wenn man
a) Oxirane der Formel

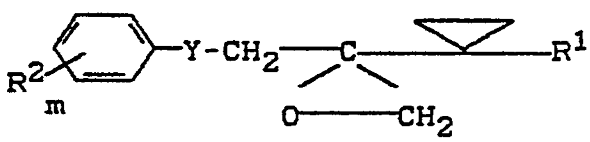

(II)

in welcher
$R^1$, $R^2$, Y und m die oben angegebene Bedeutung haben,
mit Azolen der Formel

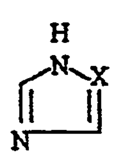

(III)

in welcher
X die oben angegebene Bedeutung hat,
in Gegenwart eines Säurebindemittels und in Gegenwart eines Verdünnungsmittels umsetzt,

oder

b) Azolyl-Derivate der Formel

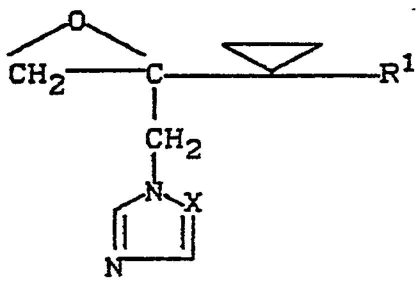

(IV)

in welcher $R^1$ und X die oben angegebene Bedeutung haben, mit Phenyl-Derivaten der Formel

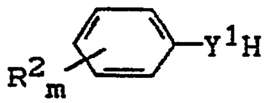

(V)

in welcher $R^2$ und m die oben angegebene Bedeutung haben und $Y^1$ für Sauerstoff oder Schwefel steht, in Gegenwart eines Säurebindemittels und in Gegenwart eines Verdünnungsmittels umsetzt,

oder

c) Hydroxyethyl-azolyl-Derivate der Formel

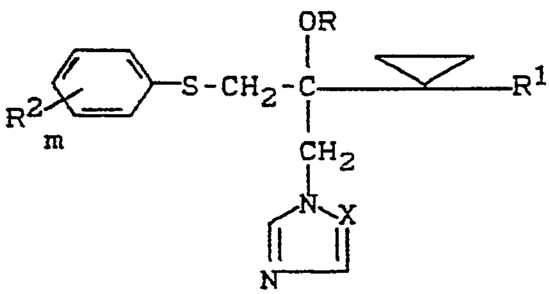

(Ia)

in welcher R, $R^1$, $R^2$, X und m die oben angegebene Bedeutung haben, mit Oxidationsmitteln gegebenenfalls in Gegenwart eines Verdünnungsmittels umsetzt,

oder

d) Hydroxyethyl-azolyl-Derivate der Formel

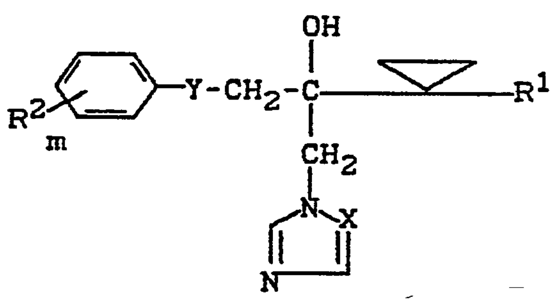

(Ib)

in welcher $R^1$, $R^2$, X, Y und m die oben angegebene Bedeutung haben, mit starken Basen in Gegenwart eines Verdünnungsmittels umsetzt und die dabei entstehenden Alkoholate der Formel

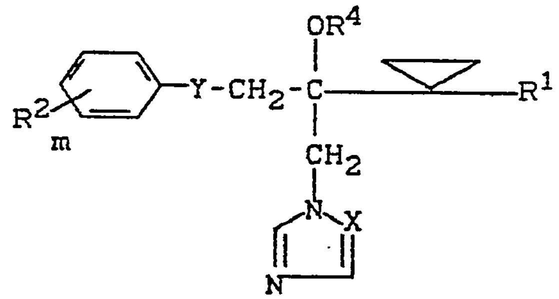

(Ic)

in welcher
$R^1$, $R^2$, X, Y und m die oben angegebene Bedeutung haben, und
$R^4$ für ein Alkalimetallkation, oder für ein quarternäres Ammonium- oder Phosphonium-kation steht,
mit Halogenverbindungen der Formel

$R^5$ – Hal (VI)

in welcher
$R^5$ für Alkyl mit 1 bis 6 Kohlenstoffatomen oder Alkylcarbonyl mit 1 bis 6 Kohlenstoffatomen in der Alkylgruppe steht
und Hal für Chlor oder Brom steht,
in Gegenwart eines Verdünnungssmittels umsetzt,
und gegebenenfalls anschliessend an die so erhaltenen Verbindungen der Formel (I) eine Säure oder ein Metallsalz addiert.

Schliesslich wurde gefunden, dass die neuen Hydroxyethylazolyl-Derivate der Formel (I) sowie deren Säureadditions-Salze und Metallsalz-Komplexe starke fungizide und pflanzenwuchsregulierende Eigenschaften besitzen.

Die erfindungsgemässen Stoffe enthalten ein asymmetrisch substituiertes Kohlenstoffatom. Sie können daher in optischen Isomerenformen anfallen. Die vorliegende Erfindung betrifft sowohl die einzelnen Isomeren als auch deren Gemische.

Überraschenderweise besitzen die erfindungsgemässen Stoffe eine bessere fungizide und pflanzenwuchsregulierende Wirksamkeit als konstitutionell ähnlichsten vorbekannten Verbindungen gleicher Wirkungsrichtung.

Die erfindungsgemässen Hydroxyethyl-azolyl-Derivate sind durch die Formel (I) allgemein definiert.

Wenn m für die Zahlen 2 oder 3 steht, können die für $R^2$ stehenden Reste gleich oder verschieden sein.

Bevorzugt sind diejenigen Verbindungen der Formel (I), in denen
R für Wasserstoff, Methyl, Ethyl, n-Propyl, Isopropyl, n-Butyl, Isobutyl, Methylcarbonyl, Ethylcarbonyl, n-Propylcarbonyl, Isopropyl-carbonyl, n-Butylcarbonyl oder Isobutyl-carbonyl steht,
$R^1$ für Fluor, Chlor, Brom, gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht oder für die Gruppierung —Z–$R^3$ steht, worin
Z für Sauerstoff, Schwefel, SO oder $SO_2$ steht und
$R^3$ für gegebenenfalls einfach bis dreifach, gleichartig oder verschieden durch Fluor, Chlor, Brom, Trifluormethyl, Methyl, Ethyl, Methoxy und/oder Ethoxy substituiertes Phenyl steht,
$R^2$ für Fluor, Chlor, Brom, Methyl, Ethyl, Isopropyl, tert.-Butyl, Methoxy, Methylthio, Trifluormethyl, Trifluormethoxy, Trifluormethylthio, Methoximinomethyl, gegebenenfalls durch Fluor, Chlor und/oder Methyl substituiertes Phenyl oder für gegebenenfalls durch Fluor, Chlor und/oder Methyl substituiertes Phenoxy steht,
m für die Zahlen 0, 1, 2 oder 3 steht,
X für Stickstoff oder eine CH-Gruppe steht und
Y für Sauerstoff, Schwefel, SO oder $SO_2$ steht.

Bevorzugte erfindungsgemässe Verbindungen sind auch Additionsprodukte aus Säuren und denjenigen Hydroxyethyl-azolyl-Derivaten der Formel (I), in denen R, $R^1$, $R^2$, X, Y und m die Bedeutungen haben, die bereits vorzugsweise für diese Reste bzw. diesen Index genannt wurden.

Zu den Säuren, die addiert werden können, gehören vorzugsweise Halogenwasserstoffsäuren, wie z.B. die Chlorwasserstoffsäure und die Bromwasserstoffsäure, insbesondere die Chlorwasserstoffsäure, ferner Phosphorsäure, Salpetersäure, Schwefelsäure, mono- und bifunktionelle Carbonsäure und Hydroxycarbonsäuren, wie z.B. Essigsäure, Maleinsäure, Bernsteinsäure, Fumarsäure, Weinsäure, Zitronensäure, Salizylsäure, Sorbinsäure und Milchsäure sowie Sulfonsäuren, wie z.B. p-Toluolsulfonsäure, 1,5-Naphthalindisulfonsäure oder Camphersulfonsäure.

Außerdem bevorzugte erfindungsgemäße Verbindungen sind Additionsprodukte aus Salzen von Metallen der II. bis IV. Haupt- und der I. und II. sowie IV. bis VIII. Nebengruppe des Periodensystems der

Elemente und denjenigen Hydroxyethyl-azolyl-Derivaten der Formel (I), in denen R, $R^1$, $R^2$, X, Y und m die Bedeutungen haben, die bereits vorzugsweise für diese Reste und diesen Index genannt wurden.

Hierbei sind die Salze des Kupfers, Zinks, Mangans, Magnesiums, Zinns, Eisens und des Nickels besonders bevorzugt. Als Anionen dieser Salze kommen solche in Betracht, die sich von solchen Säuren ableiten, die zu physiologisch verträglichen Additionsprodukten führen. Besonders bevorzugte derartige Säuren sind in diesem Zusammenhang die Halogenwasserstoffsäuren, wie z.B. die Chlorwasserstoffsäure und die Bromwasserstoffsäure, ferner Phosphorsäure, Salpetersäure und Schwefelsäure.

Als Beispiele für Hydroxyethyl-azolyl-Derivate der Formel (I) seien die in der folgenden Tabelle aufgeführten Stoffe genannt.

Tabelle 1

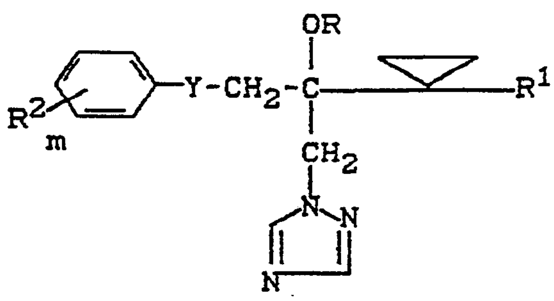

(I)

| $R^2_m$ | R | X | $R^1$ | Y |
|---|---|---|---|---|
| 2,4-$Cl_2$ | H | N | Cl | O |
| 2,4-$F_2$ | H | N | Cl | O |
| 4-$CH_3$ | H | N | Cl | O |
| 4-$CF_3$ | H | N | Cl | O |
| 4-$OCF_3$ | H | N | Cl | O |
| 4-$OCH_3$ | H | N | Cl | O |
| 4-$SCH_3$ | H | N | Cl | O |
| 2,4,6-$Cl_3$ | H | N | Cl | O |
| 4-Cl | H | N | F | O |
| 4-Cl | H | CH | Cl | O |
| 4-Cl | $CH_3$ | N | Cl | O |
| 4-Cl | H | N | 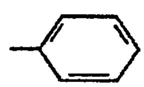 | O |
| 4-Cl | H | N | 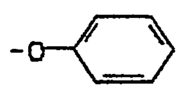 | O |
| 4-Cl | H | N | 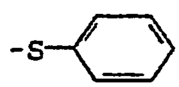 | O |
| 4-Cl | H | N | 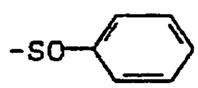 | O |
| 4-Cl | H | N | 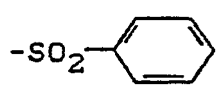 | O |
| 4 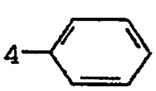 | H | N | Cl | O |

Tabelle 1 (Fortsetzung)

| $R^2_m$ | R | X | $R^1$ | Y |
|---|---|---|---|---|
| 4-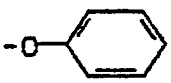 | H | N | Cl | O |
| 4-$C_4H_9$-t. | H | N | Cl | O |
| 2-Cl,4-$CH_3$ | H | N | Cl | O |
| | H | N | Cl | O |
| 4-Cl | -CO-$CH_3$ | N | Cl | O |
| 4-Cl | -$C_2H_5$ | N | Cl | O |
| 4-F | $CH_3$ | N | F | O |
| 2,4-$Cl_2$ | H | N | Cl | S |
| 2,4-$F_2$ | H | N | Cl | S |
| 4-$CH_3$ | H | N | Cl | S |
| 4-$CF_3$ | H | N | Cl | S |
| 4-$OCF_3$ | H | N | Cl | S |
| 4-$OCH_3$ | H | N | Cl | S |
| 4-$SCH_3$ | H | N | Cl | S |
| 2,4,6-$Cl_3$ | H | N | Cl | S |
| 4-Cl | H | N | F | S |
| 4-Cl | H | CH | Cl | S |
| 4-Cl | $CH_3$ | N | Cl | S |
| 4-Cl | H | N | 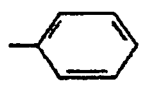 | S |
| 4-Cl | H | N | 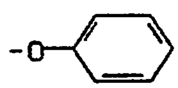 | S |
| 4-Cl | H | N | 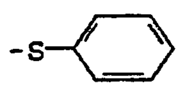 | S |
| 4-Cl | H | N | 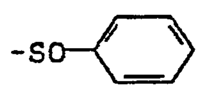 | S |
| 4-Cl | H | N | 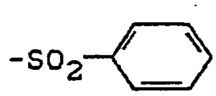 | S |
| 4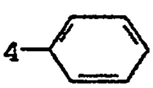 | H | N | Cl | S |

Tabelle 1 (Fortsetzung)

| $R^2_m$ | R | X | $R^1$ | Y |
|---|---|---|---|---|
| 4-O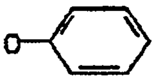 | H | N | Cl | S |
| 4-$C_4H_9$-t. | H | N | Cl | S |
| 2-Cl,4-$CH_3$ | H | N | Cl | S |
| - | H | N | Cl | S |
| 4-Cl | -CO-$CH_3$ | N | Cl | S |
| 4-Cl | -$C_2H_5$ | N | Cl | S |
| 4-F | $CH_3$ | N | F | S |
| 2,4-$Cl_2$ | H | N | Cl | SO |
| 2,4-$F_2$ | H | N | Cl | SO |
| 4-$CH_3$ | H | N | Cl | SO |
| 4-$CF_3$ | H | N | Cl | SO |
| 4-$OCF_3$ | H | N | Cl | SO |
| 4-$OCH_3$ | H | N | Cl | SO |
| 2,4,6-$Cl_3$ | H | N | Cl | SO |
| 4-Cl | H | N | F | SO |
| 4-Cl | H | CH | Cl | SO |
| 4-Cl | $CH_3$ | N | Cl | SO |
| 4-Cl | H | N | 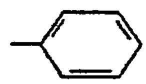 | SO |
| 4-Cl | H | N | -O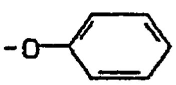 | SO |
| 4-Cl | H | N | -SO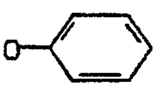 | SO |
| 4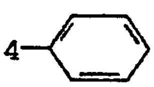 | H | N | Cl | SO |

Tabelle 1 (Fortsetzung)

| $R^2_m$ | R | X | $R^1$ | Y |
|---|---|---|---|---|
| 4-O 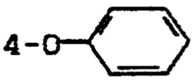 | H | N | Cl | SO |
| $4-C_4H_9$-t. | H | N | Cl | SO |
| $2-Cl,4-CH_3$ | H | N | Cl | SO |
| - | H | N | Cl | SO |
| 4-Cl | $-CO-CH_3$ | N | Cl | SO |
| 4-Cl | $-C_2H_5$ | N | Cl | SO |
| 4-F | $CH_3$ | N | F | SO |
| $2,4-Cl_2$ | H | N | Cl | $SO_2$ |
| $2,4-F_2$ | H | N | Cl | $SO_2$ |
| $4-CH_3$ | H | N | Cl | $SO_2$ |
| $4-CF_3$ | H | N | Cl | $SO_2$ |
| $4-OCF_3$ | H | N | Cl | $SO_2$ |
| $4-OCH_3$ | H | N | Cl | $SO_2$ |
| $2,4,6-Cl_3$ | H | N | Cl | $SO_2$ |
| 4-Cl | H | N | F | $SO_2$ |
| 4-Cl | H | CH | Cl | $SO_2$ |
| 4-Cl | $CH_3$ | N | Cl | $SO_2$ |
| 4-Cl | H | N | 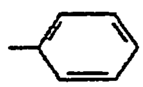 | $SO_2$ |
| 4-Cl | H | N | -O 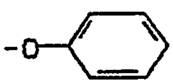 | $SO_2$ |
| 4-Cl | H | N | $-SO_2$ 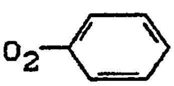 | $SO_2$ |
| 4 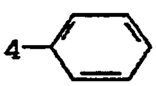 | H | N | Cl | $SO_2$ |
| 4-O 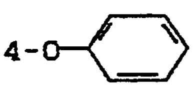 | H | N | Cl | $SO_2$ |
| $4-C_4H_9$-t. | H | N | Cl | $SO_2$ |
| $2-Cl,4-CH_3$ | H | N | Cl | $SO_2$ |
| - | H | N | Cl | $SO_2$ |
| 4-Cl | $-CO-CH_3$ | N | Cl | $SO_2$ |
| 4-Cl | $-C_2H_5$ | N | Cl | $SO_2$ |
| 4-F | $CH_3$ | N | F | $SO_2$ |

Verwendet man 2-(1-Chlor-cyclopropyl)-2-(4-chlorphenoxymethyl)-oxiran und 1,2,4-Triazol als Ausgangsstoffe, so kann der Verlauf des erfindungsgemässen Verfahrens (a) durch das folgende Formelschema veranschaulicht werden:

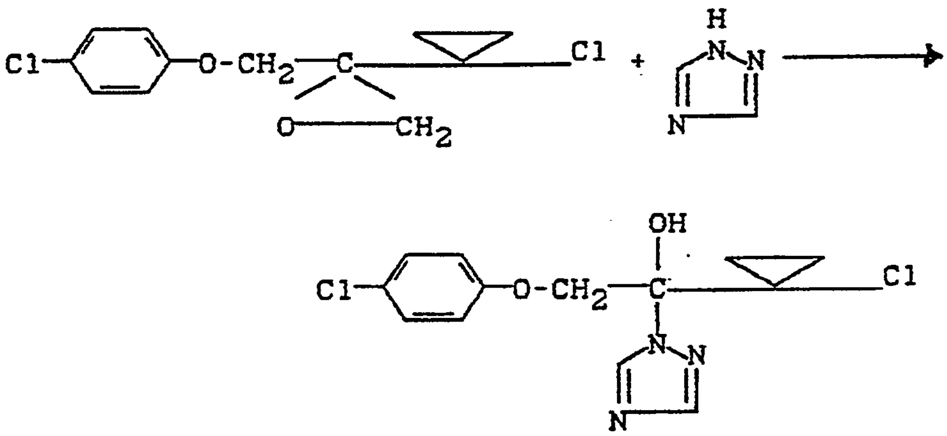

Verwendet man 2-[1-(2-Chlorphenoxy)-cyclopropyl]-2-[(1,2,4-triazol-1-yl)-methyl]-oxiran und 4-Chlor-thiophenol als Ausgangsstoffe, so kann der Verlauf des erfindungsgemässen Verfahrens (b) durch das folgende Formelschema veranschaulicht werden:

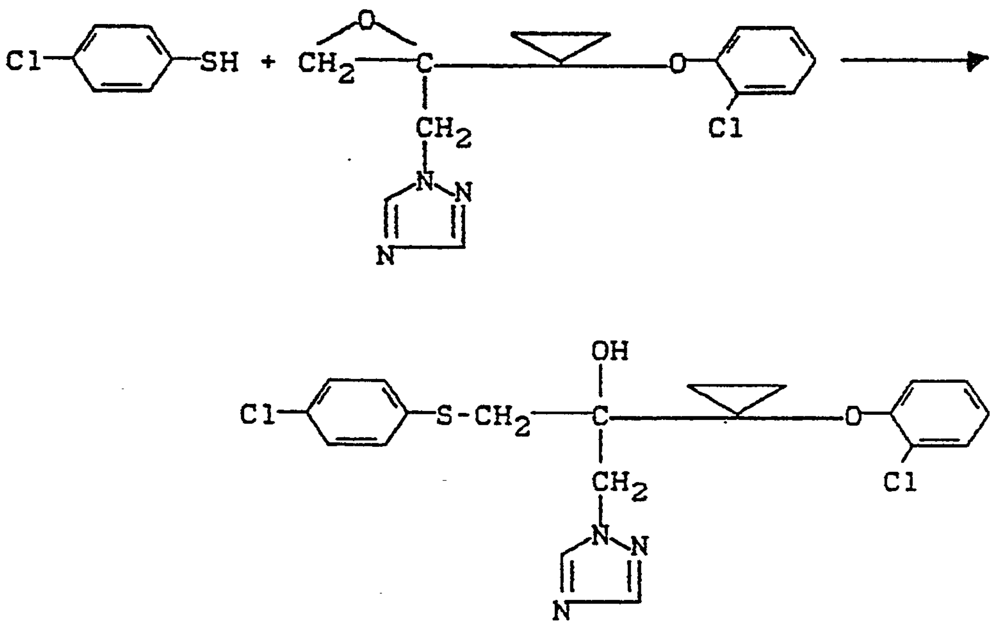

Verwendet man 1-(4-Chlorphenylmercapto)-2-[1-(2-chlorphenoxy)-cyclopropyl]-3-(1,2,4-triazol-1-yl)-propan-2-ol als Ausgangsstoff und wässriges Wasserstoffperoxid in Eisessig als Oxidationsmittel, so kann der Verlauf des erfindungsgemässen Verfahrens (c) durch das folgende Formelschema veranschaulicht werden:

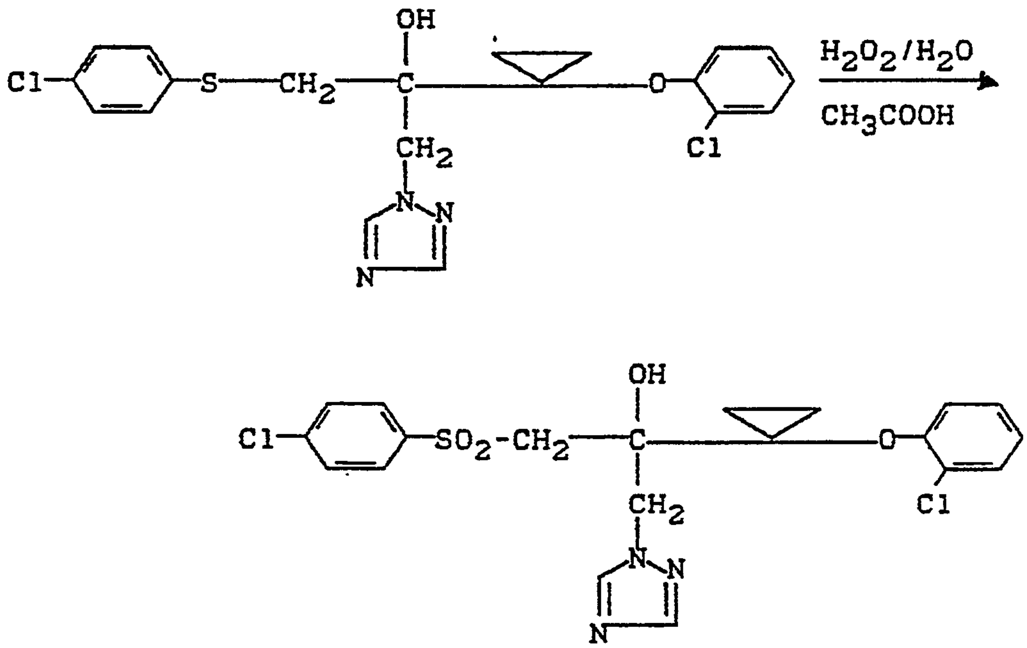


Verwendet man 2-(1-Chlorcyclopropyl)-1-(4-chlorphenoxy)-3-(1,2,4-triazol-1-yl)-propan-2-ol und Natriumhydrid als Ausgangsstoffe und Jodmethan als Reaktionskomponente, so kann der Verlauf des erfindungsgemässen Verfahrens (d) durch das folgende Formelschema veranschaulicht werden:

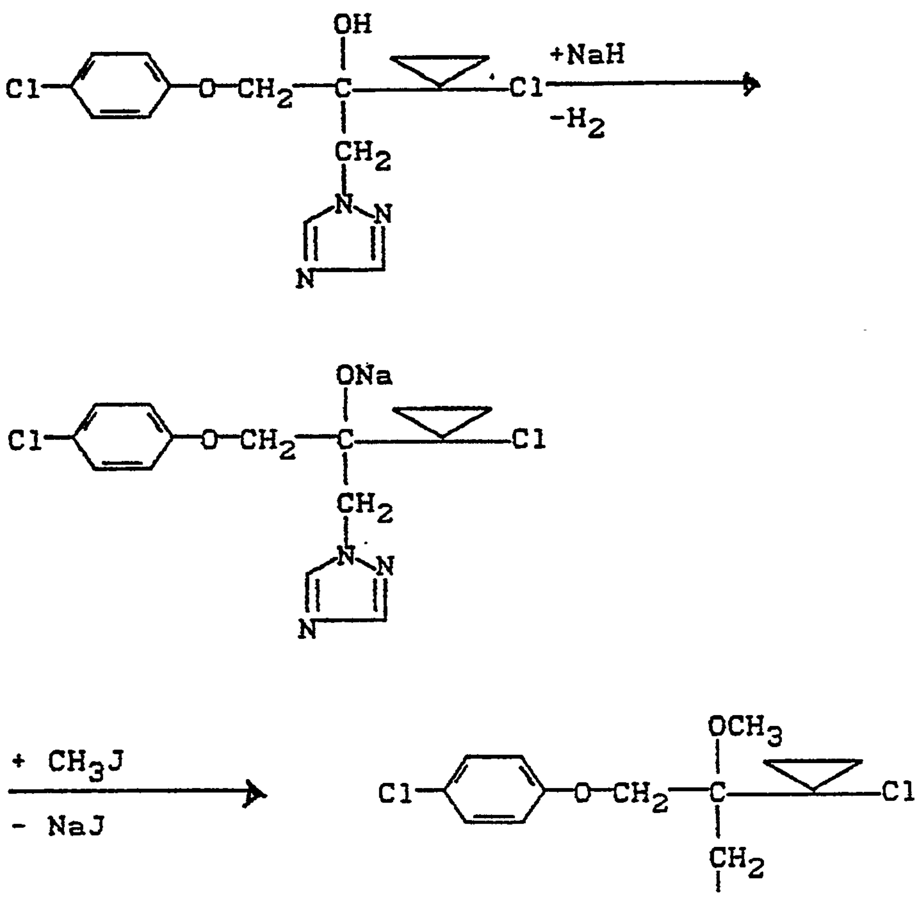

Die bei dem erfindungsgemässen Verfahren (a) als Ausgangsstoffe benötigten Oxirane sind durch die Formel (II) allgemein definiert. In dieser Formel haben $R^1$, $R^2$, Y und m vorzugsweise diejenigen Bedeutungen, die bereits im Zusammenhang mit der Beschreibung der erfindungsgemäßen Stoffe der Formel (I) vorzugsweise für diese Reste und für diesen Index genannt wurden.

Die Oxirane der Formel (II) sind bisher noch nicht bekannt. Sie lassen sich herstellen, indem man

e) Cyclopropylketone der Formel

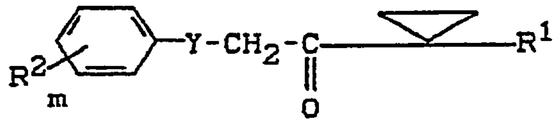

(VII)

in welcher

$R^1$, $R^2$, Y und m die oben angegebene Bedeutung haben,

entweder

α) mit Dimethyloxosulfonium-methylid der Formel

$$\overset{\delta^{\ominus}\delta^{\ominus}}{(CH_3)_2SOCH_2} \qquad (VIII)$$

oder

β) mit Dimethylsulfonium-methylid der Formel

$$\overset{\delta^{\ominus}}{(CH_3)_2S}\ \ \overset{\delta^{\ominus}}{CH_2} \qquad (IX)$$

in Gegenwart eines Verdünnungsmittels umsetzt.

Die bei dem Verfahren (e) als Ausgangsstoffe benötigten Cyclopropylketone der Formel (VII) sind ebenfalls noch nicht bekannt. Sie lassen sich herstellen, indem man

f) Halogenketone der Formel

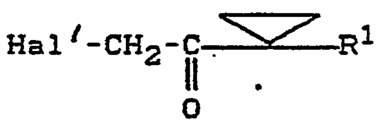

(X)

in welcher

$R^1$ die oben angegebene Bedeutung hat und Hal' für Chlor oder Brom steht, mit Phenyl-Derivaten der Formel

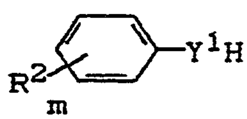

(V)

in welcher

$R^2$, $Y^1$ und m die oben angegebene Bedeutung haben, in Gegenwart eines Säurebindemittels und gegebenenfalls in Gegenwart eines Verdünnungsmittels umsetzt und gegebenenfalls anschliessend oxidiert.

Die bei dem Verfahren (f) als Ausgangsstoffe benötigten Halogenketone der Formel (X) sind teilweise bekannt. Sie lassen sich herstellen, indem man

g) Ketone der Formel

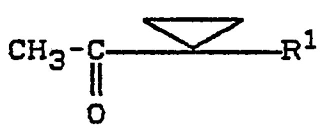

(XI)

in welcher

$R^1$ die oben angegebene Bedeutung hat, mit Chlorierungsmitteln oder Bromierungsmitteln in Gegenwart eines Verdünnungsmittels umsetzt.

Die bei dem Verfahren (g) als Ausgangsstoffe benötigten Ketone der Formel (XI) sind bekannt oder lassen sich nach prinzipiell bekannten Verfahren synthetisieren (vgl. Synthesis 1977, 189).

Als Chlorierungsmittel und Bromierungsmittel kommen bei dem Verfahren (g) alle für derartige Umsetzungen üblichen Chlorierungs- und Bromierungsreagenzien in Betracht. Vorzugsweise verwendbar sind Sulfurylchlorid, Sulfurylbromid, Chlor und Brom.

Als Verdünnungsmittel kommen bei dem Verfahren (g) alle für derartige Umsetzungen üblichen inerten organischen Solventien in Frage. Vorzugsweise verwendbar sind halogenierte aliphatische Kohlenwasserstoffe, wie Methylenchlorid, Chloroform und Tetrachlorkohlenstoff.

Die Reaktionstemperaturen können bei dem Verfahren (g) innerhalb eines bestimmten Bereiches variiert werden. Im allgemeinen arbeitet man bei Temperaturen zwischen –10°C und +60°C, vorzugsweise zwischen 0°C und +40°C.

Bei der Durchführung des Verfahrens (g) arbeitet man ebenso wie bei den übrigen in dieser Anmeldung beschriebenen Verfahren im allgemeinen unter Normaldruck. Es ist jedoch jeweils auch möglich, unter erhöhtem oder vermindertem Druck zu arbeiten.

Bei der Durchführung des Verfahrens (g) setzt man auf 1 Mol an Keton der Formel (XI) im allgemeinen eine stöchiometrische Menge oder auch einen geringen Überschuss an Chlorierungs- oder Bromierungsmittel ein. Die Aufarbeitung erfolgt nach üblichen Methoden. Im allgemeinen geht man so vor, daß man das Reaktionsgemisch nacheinander mit verdünnter, wässriger Natriumhydrogencarbonat-Lösung und Wasser wäscht, dann trocknet und einengt.

Die bei dem Verfahren (f) als Reaktionskomponenten benötigten Phenyl-Derivate sind durch die Formel (V) allgemein definiert. In dieser Formel haben $R^2$, $Y^1$ und m vorzugsweise diejenigen Bedeutungen, die bereits im Zusammenhang mit der Beschreibung der erfindungsgemässen Stoffe der Formel (I) vorzugsweise für diese Reste und für diesen Index genannt wurden.

–Die Phenyl-Derivate der Formel (V) sind allgemein bekannte Verbindungen der organischen Chemie.

Als Säurebindemittel kommen bei der Durchführung des Verfahrens (f) alle üblichen Säureakzeptoren in Betracht, Vorzugsweise verwendbar sind Alkalimetallcarbonate und Hydrogencarbonate, wie Natriumcarbonat, Kaliumcarbonat oder Natriumhydrogencarbonat, ferner Alkalimetallhydroxide und -Alkoholate, wie Natriumhydroxid, Kaliumhydroxid, Natriummethylat oder Kalium-tert.-butylat, ausserdem tertiäre aliphatische oder aromatische Amine, wie Triethylamin, N,N-Dimethyl-cyclohexyl-amin, N,N-Dimethyl-benzylamin und Pyridin, und ausserdem cyclische Amine, wie 1, 5-Diaza-bicyclo [4.3.0]non-5-en (DBN), 1,8-Diaza-bicyclotH5.4.0]undec-7en (DBU) und 1,4-Diaza-bicyclo[2.2.2]octan (DABCO).

Als Verdünnungsmittel kommen bei der Durchführung des Verfahrens (f) alle inerten organischen Solventien in Betracht. Vorzugsweise verwendbar sind aliphatische und aromatische, gegebenenfalls halogenierte Kohlenwasserstoffe wie Pentan, Hexan, Heptan, Cyclohexan, Petrolether, Benzin, Ligroin, Benzol, Toluol, Xylol, Methylenchlorid, Ethylenchlorid, Chloroform, Tetrachlorkohlenstoff, Chlorbenzol und o-Dichlorbenzol, Ether wie Dimethyl- und Dibutylether, Glykoldimethylether und Diglykoldimethylether, Tetrahydrofuran und Dioxan, Ketone wie Aceton, Methylethyl-, Methyl-isopropyl- und Methylisobutyl-keton, Ester wie Essigsäuremethylester und -ethylester, Nitrile wie z.B. Acetonitril und Propionitril und Pyridin, sowie auch stark polare Lösungsmittel, wie Dimethylformamid und Dimethylsulfoxid.

Die Reaktionstemperaturen können bei dem Verfahren (f) innerhalb eines gröBeren Bereiches variiert werden. Im allgemeinen arbeitet man bei Temperaturen zwischen 0°C und 150°C, vorzugsweise zwischen 20°C und 130°C.

Bei der Durchführung des Verfahrens (f) setzt man auf 1 Mol an Halogenketon der Formel (X) im allgemeinen 1 bis 1,5 Mol an Phenyl-Derivat der Formel (V) sowie 1 bis 2 Mol an Säurebindemittel ein. Die Aufarbeitung erfolgt nach üblichen Methoden. Im allgemeinen geht man so vor, dass man gegebenenfalls nach vorherigem Abfiltrieren von abgeschiedenen Salzen das Reaktionsgemisch einengt, den Rückstand in einem mit Wasser wenig mischbaren organischen Lösungsmittel aufnimmt, die entstehende Lösung wäscht, trocknet und dann einengt.

Das bei dem Verfahren (e) als Reaktionskomponente benötigte Dimethyl-oxo-sulfonium-methylid der Formel (VIII) ist bekannt (vgl. J. Am. Chem. Soc. 87, 1363–1364 (1965)). Es wird bei der obigen Umsetzung in frisch zubereitetem Zustand verarbeitet, indem man es in situ durch Umsetzung von Trimethyloxosulfoniumiodid mit Natriumhydrid oder Natriumamid, Kalium-tert.-butylat oder Natriummethylat, in Gegenwart eines Verdünnungsmittels erzeugt.

Das bei dem Verfahren (e) ausserdem als Reaktionskomponente in Betracht kommende Dimethylsulfonium-methylid der Formel (IX) ist ebenfalls bekannt (vgl. Heterocycles 8, 397 (1977)). Es wird bei der obigen Umsetzung ebenfalls in frisch hergestelltem Zustand eingesetzt, indem man es in situ zum Beispiel aus Trimethylsulfoniumhalogenid oder Trimethylsulfoniummethylsulfat, in Gegenwart einer starken Base, wie zum Beispiel Natriumhydrid, Natriumamid, Natriummethylat, Kalium-tert.-butylat oder Kaliumhydroxid, in Gegenwart eines Verdünnungsmittels, wie tert.-Butanol oder Dimethylsulfoxid erzeugt.

Als Verdünnungsmittel kommen bei der Durchführung des Verfahrens (e) inerte organische Solventien in Frage. Vorzugsweise verwendbar sind Alkohole, wie tert.-Butanol, Ether, wie Tetrahydrofuran oder Dioxan, ferner aliphatische und aromatische Kohlenwasserstoffe, wie Benzol, Toluol oder Xylol, sowie stark polare Lösungsmittel, wie Dimethylsulfoxid oder Acetonitril.

Die Reaktionstemperaturen können bei der Durchführung des Verfahrens (e) in einem grösseren Bereich variiert werden, Im allgemeinen arbeitet man zwischen 0°C und 100°C, vorzugsweise zwischen 10°C und 60°C.

Bei der Durchführung des Verfahrens (e) setzt man auf 1 Mol an Cyclopropyl-keton der Formel (VII) im allgemeinen 1 bis 3 Mol an Dimethyloxosulfonium-methylid der Formel (VIII) bzw. an Dimethylsulfonium-methylid der Formel (IX) ein. Die Isolierung der Oxirane der Formel (II) erfolgt nach üblichen Methoden.

Als Säurebindemittel kommen bei der Durchführung des erfindungsgemässen Verfahrens (a) alle üblichen Säureakzeptoren in Frage. Vorzugsweise verwendbar sind alle diejenigen Säurebindemittel, die bereits im Zusammenhang mit der Beschreibung des Verfahrens (f) vorzugsweise genannt wurden.

Als Verdünnungsmittel kommen bei der Durchführung des erfindungsgemässen Verfahrens (a) alle üblichen inerten organischen Solventien in Betracht.

Vorzugsweise verwendbar sind Nitrile, wie insbesondere Acetonitril; aromatische Kohlenwasserstoffe, wie Benzol, Toluol und Dichlorbenzol; Formamide, wie insbesondere Dimethylformamid, sowie Hexamethylphosphorsäuretriamid.

Die Reaktionstemperaturen können bei der Durchführung des erfindungsgemässen Verfahrens (a) in einem größeren Bereich variiert werden. Im allgemeinen arbeitet man bei Temperaturen zwischen 0°C und 200°C, vorzugsweise zwischen 50°C und 150°C.

Bei der Durchführung des erfindungsgemässen Verfahrens (a) setzt man vorzugsweise auf 1 Mol an Oxiran der Formel (II), 1 bis 4 Mol an Azol der Formel (III) und 1 bis 2 Mol an Base ein. Die Isolierung der Endprodukte erfolgt in üblicher Weise.

Die bei der Durchführung des erfindungsgemässen Verfahrens (b) als Ausgangsstoffe benötigten Azolyl-Derivate sind durch die Formel (IV) allgemein definiert. In dieser Formel haben $R^1$ und X vorzugsweise diejenigen Bedeutungen, die bereits im Zusammenhang mit der Beschreibung der erfindungsgemässen Stoffe der Formel (I) vorzugsweise für diese Reste genannt wurden.

Die Azolyl-Derivate der Formel (IV) sind bisher noch nicht bekannt, Sie lassen sich herstellen, indem man

h) Azolylketone der Formel

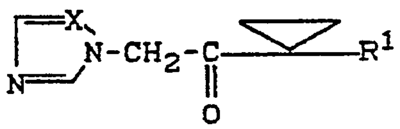 (XII)

in welcher $R^1$ und X die oben angegebene Bedeutung haben,
entweder
α) mit Dimethyloxosulfonium-methylid der Formel

$$(CH_3)_2\overset{\delta\oplus}{SO}\ \overset{\delta\ominus}{CH_2} \qquad (VIII)$$

oder
β) mit Dimethylsulfonium-methylid der Formel

$$(CH_3)_2\overset{\delta\oplus}{S}\ \overset{\delta\ominus}{CH_2} \qquad (IX)$$

in Gegenwart eines Verdünnungsmittels umsetzt.

Die bei der Durchführung des Verfahrens (h) als Ausgangsstoffe benötigten Azolylketone sind ebenfalls noch nicht bekannt. Sie lassen sich herstellen, indem man

i) Halogenketone der Formel

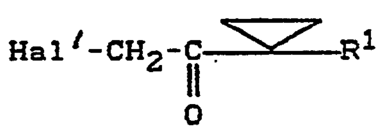 (X)

in welcher
$R^1$ die oben angegebene Bedeutung hat und Hal' für Chlor oder Brom steht, mit Azolen der Formel

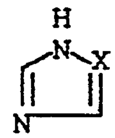

(III)

in welcher

X die oben angegebene Bedeutung hat, in Gegenwart eines Säurebindemittels und in Gegenwart eines Verdünnungsmittels umsetzt.

Bei der Durchführung des Verfahrens (i) entsprechen die Reaktionsbedingungen denjenigen des Verfahrens (f).

Die Durchführung des Verfahrens (h) erfolgt analog zu derjenigen des Verfahrens (e).

Die bei der Durchführung des erfindungsgemässen Verfahrens (b) als Reaktionskomponenten benötigten Phenyl-Derivate wurden bereits im Zusammenhang mit der Beschreibung des Verfahrens (f) abgehandelt.

Bei der Durchführung des erfindungsgemässen Verfahrens (b) können alle üblichen Säurebindemittel und Verdünnungsmittel eingesetzt werden. Vorzugsweise verwendbar sind diejenigen Säurebindemittel und Lösungsmittel, die bereits im Zusammenhang mit der Beschreibung des Verfahrens (a) vorzugsweise genannt wurden.

Die Reaktionstemperaturen können auch bei der Durchführung des erfindungsgemässen Verfahrens (b) in einem grösseren Bereich variiert werden. Im allgemeinen arbeitet man bei Temperaturen zwischen 0 und 200°C, vorzugsweise zwischen 50 und 150°C.

Bei der Durchführung des erfindungsgemässen Verfahrens (b) setzt man auf 1 Mol Azolyl-Derivat der Formel (IV) im allgemeinen eine äquivalente Menge oder auch einen Überschuss an Phenyl-Derivat der Formel (V) sowie 2 bis 4 Mol an Säurebindemittel ein. Die Aufarbeitung erfolgt nach üblichen Methoden. Im allgemeinen geht man so vor, daß man das Reaktionsgemisch filtriert, das Filtrat durch Abziehen des Verdünnungsmittels einengt und den verbleibenden Rückstand in üblicher Weise reinigt.

Die für das erfindungsgemässe Verfahren (c) als Ausgangsstoffe benötigten Hydroxyethyl-azolyl-Derivate der Formel (Ia) sind erfindungsgemäße Verbindungen.

Als Reaktionskomponenten kommen bei dem erfindungsgemässen Verfahren (c) alle für derartige Umsetzungen üblichen Oxidationsmittel in Betracht. Vorzugsweise verwendbar sind Wasserstoffperoxid und Persäuren, wie m-Chlorperbenzoesäure und Peressigsäure.

Bei der Durchführung des erfindungsgemäßen Verfahrens (c) setzt man auf 1 Mol der erfindungsgemäßen Verbindungen der Formel (Ia) etwa 1 bis 5 Mol Oxidationsmittel ein. Bei der Anwendung von 1 Mol Oxidationsmittel, wie m-Chlorperbenzoesäure in Methylenchlorid oder Wasserstoffperoxid in Essigsäure oder Acetanhydrid bei Temperaturen zwischen –30°C bis +90°C, entstehen vorzugsweise die erfindungsgemäßen Verbindungen der Formel (I), in denen Y für eine –SO-Gruppierung steht. Bei ÜberschuB an Oxidationsmittel und Temperaturen zwischen 10°C und 90°C entstehen vorzugsweise die erfindungsgemäßen Verbindungen der Formel (I), in denen Y für eine $-SO_2$-Gruppierung steht, Die Isolierung der Oxidationsprodukte erfolgt in üblicher Weise.

Die für das erfindungsgemaße Verfahren (d) aus Ausgangsstoff benötigten Hydroxyethyl-azolyl-Derivate der Formel (Ib) sind ebenfalls erfindungsgemaße Verbindungen. Ihre Überführung in die entsprechenden Alkoholate erfolgt in allgemein bekannter Weise, indem man mit geeigneten starken Basen, wie Alkalimetall-amiden oder -hydriden, quarternären Ammonium-hydroxiden oder Phosphonium-hydroxiden in einem inerten Verdünnungsmittel, wie zum Beispiel Dioxan, bei Raumtemperatur umsetzt. Demgemäß steht $R^4$ in den Verbindungen der Formel (Ic) für ein Alkalimetallkation, wie ein Natrium- oder Kalium-kation, oder für ein quarternäres Ammonium- oder Phosphonium-kation.

Die bei dem erfindungsgemäßen Verfahren (d) ausserdem als Ausgangsstoffe benötigten Halogenverbindungen sind durch die Formel (VI) allgemein definiert. In dieser Formel steht $R^5$ für die Bedeutungen, die bereits im Zusammenhang mit der Beschreibung der erfindungsgemäßen Stoffe der Formel (I) für den Substituenten R genannt wurden, mit Ausnahme der Bedeutung von Wasserstoff. Hal steht vorzugsweise für Chlor oder Brom.

Die Halogenverbindungen der Formel (VI) sind bekannt oder lassen sich nach im Prinzip bekannten Methoden herstellen.

Als Verdünnungsmittel kommen bei der Durchführung des erfindungsgemäßen Verfahrens (d) inerte organische Lösungsmittel in Frage. Hierzu gehören vorzugsweise Ether, wie Diethylether oder Dioxan; aromatische Kohlenwasserstoffe, wie Benzol; in einzelnen Fällen auch chlorierte Kohlenwasserstoffe, wie Chloroform, Methylenchlorid oder Tetrachlorkohlenstoff; sowie Hexamethylphosphorsäuretriamid.

Die Reaktionstemperaturen können bei der Durchfuhrung des erfindungsgemäßen Verfahrens (d) in einem größeren Bereich variiert werden. Im allgemeinen arbeitet man zwischen 0°C und 120°C, vorzugsweise zwischen 20°C und 100°C.

Bei der Durchführung des erfindungsgemäßen Verfahrens (d) setzt man zunächst Hydroxy-Verbindungen der Formel (Ib) mit starken Basen zu den entsprechenden Alkoholaten der Formel (Ic) um. In der danach folgenden Stufe setzt man auf 1 Mol eines Alkoholates der Formel (Ic) vorzugsweise 1 bis 2 Mol Halogenverbindung der Formel (VI) ein.

Zur Isolierung der Endprodukte wird das Reaktionsgemisch vom Lösungsmittel befreit und der Rückstand mit Wasser und einem organischen Lösungsmittel versetzt. Die organische Phase wird abgetrennt, in üblicher Weise aufgearbeitet und gereinigt.

In einer bevorzugten Ausführungsform wird zweckmäßigerweise so verfahren, daß man von einer Hydroxy-Verbindung der Formel (Ib) ausgeht, letztere in einem geeigneten organischen Lösungsmittel mittels Alkalimetall-hydrid oder Alkalimetall-amid in das Alkalimetallalkoholat überführt und letzteres ohne Isolierung sofort mit einer Halogenverbindung der Formel (VI) umsetzt, wobei unter Austritt von Alkalimetallhalogenid die erfindungsgemässen Verbindungen der Formel (I) in einem Arbeitsgang erhalten werden.

Nach einer weiteren bevorzugten Ausführungsform werden zweckmässigerweise die Herstellung der Alkohole sowie die Umsetzung mit einer Halogenverbindung der Formel (VI) in einem Zweiphasensystem, wie beispielsweise wässrige Natron- oder Kalilauge/Toluol oder Methylenchlorid, unter Zusatz von 0,01-1 Mol eines Phasen-Transfer-Katalysators, wie beispielsweise Ammonium- oder Phosphoniumverbindungen, durchgeführt, wobei in der organischen Phase oder an der Grenzfläche die Alkoholate mit den in der organischen Phase befindlichen Halogeniden umgesetz werden.

Die nach den erfindungsgemäßen Verfahren erhältlichen Hydroxyethyl-azolyl-Derivate der Formel (I) können in Säureadditions-Salze bzw. Metallsalz-Komplexe überführt werden.

Zur Herstellung von Säureadditions-Salzen der Verbindungen der Formel (I) kommen vorzugsweise diejenigen Säuren in Frage, die bereits im Zusammenhang mit der Beschreibung der erfindungsgemäßen Säureadditions-Salze als bevorzugte Säuren genannt wurden.

Die Säureadditions-Salze der Verbindungen der Formel (I) können in einfacher Weise nach üblichen Salzbildungsmethoden, z.B. durch Lösen einer Verbindung der Formel (I) in einem geeigneten inerten Lösungsmittel und Hinzufügen der Säure, z.B. Chlorwasserstoffsäure, erhalten werden und in bekannter Weise, z.B. durch Abfiltrieren, isoliert und gegebenenfalls durch Waschen mit einem inerren organischen Lösungsmittel gereinigt werden.

Zur Herstellung von Metallsalz-Komplexen der Verbindungen der Formel (I) kommen vorzugsweise diejenigen Salze von Metallen in Frage, die bereits im Zusammenhang mit der Beschreibung der erfindungsgemäßen Metallsalz-Komplexe als bevorzugte Metallsalze genannt wurden.

Die Metallsalz-Komplexe der Verbindungen der Formel (I) können in einfacher Weise nach üblichen Verfahren erhalten werden, so z.B. durch Lösen des Metallsalzes in Alkohol, z.B. Ethanol und Hinzufügen zu Verbindungen der Formel (I). Man kann Metallsalz-Komplexe in bekannter Weise, z.B. durch Abfiltrieren, isolieren und gegebenenfalls durch Umkristallisation reinigen.

Die erfindungsgemäßen Wirkstoffe weisen eine starke mikrobizide Wirkung auf und können als Fungizide eingesetzt werden.

Fungizide werden im Pflanzenschutz eingesetzt zur Bekämpfung von Plasmodiophoromycetes, Oomycetes, Chytridiomycetes, Zygomycetes, Ascomycetes, Basidiomycetes, Deuteromycetes.

Beispielhaft aber nicht begrenzend seien einige Erreger von pilzlichen und bakteriellen Erkrankungen, die unter die oben aufgezählten Oberbegriffe fallen, genannt:

Xanthomonas-Arten, wie Xanthomonas oryzae; Pseudomonas-Arten, wie Pseudomonas lachrymans; Erwinia-Arten, wie Erwinia amylovora; Pythium-Arten, wie Pythium ultimum; Phytophthora-Arten, wie Phytophthora infestans; Pseudoperonospora-Arten, wie Pseudoperonospora humuli oder Pseudoperonospora cubense; Plasmopara-Arten, wie Plasmopara viticola; Peronospora-Arten, wie Peronospora pisi oder P. brassicae; Erysiphe-Arten, wie Erysiphe graminis; Sphaerotheca-Arten, wie Sphaerotheca fuliginea; Podosphaera-Arten, wie Podosphaera leucotricha; Venturia-Arten, wie Venturia inaequalis; Pyrenophora-Arten, wie Pyrenophora teres oder P. graminea; (Konidienform: Drechslera, Syn: Helminthosporium; Cochliobolus-Arten, wie Cochliobolus sativus; (Konidienform: Drechslera, Syn: Helminthosporium); Uromyces-Arten, wie Uromyces appendiculatus; Puccinia-Arten, wie Puccinia recondita; Tilletia-Arten, wie Tilletia caries; Ustilago-Arten, wie Ustilago nuda oder Ustilago avenae; Pellicularia-Arten, wie Pellicularia sasakii; Pyricularia-Arten, wie Pyricularia oryzae; Fusarium-Arten, wie Fusarium culmorum; Botrytis-Arten, wie Botrytis cinerea; Septoria-Arten, wie Septoria nodorum; Leptosphaeria-Arten, wie Leptosphaeria nodorum; Cercospora-Arten, wie Cercospora canescens; Alternaria-Arten, wie Alternaria brassicae; Pseudocercosporella-Arten, wie Pseudocercosporella herpotrichoides.

Die gute Pflanzenverträglichkeit der Wirkstoffe in den zur Bekämpfung von Pflanzenkrankheiten notwendigen Konzentrationen erlaubt eine Behandlung von oberirdischen Pflanzenteilen, von Pflanz- und Saatgut und des Bodens.

Die erfindungsgemäßen Wirkstoffe eignen sich insbesondere zur Bekampfung von Getreide- und Reiskrankheiten, wie Rost, Mehltau, Cochliobolus sativus, Pyrenophora teres und Leptosphaeria nodorum an Getreide, oder Pyricularia oryzae an Reis. Ferner lassen sie sich gegen Sphaerotheca an Gurken verwenden.

Außerdem besitzen die erfindungsgemäßen Wirkstoffe auch pflanzenwuchsregulierende Eigenschaften.

Die erfindungsgemäßen Wirkstoffe greifen in den Metabolismus der Pflanzen ein und können deshalb als Wachstumsregulatoren eingesetzt werden.

Für die Wirkungsweise von Pflanzenwachstumsregulatoren gibt nach der bisherigen Erfahrung, daß ein Wirkstoff auch mehrere verschiedenartige Wirkungen auf Pflanzen ausüben kann. Die Wirkungen der Stoffe hängen im wesentlichen ab von dem Zeitpunkt der Anwendung bezogen auf das Entwicklungsstadium der Pflanze sowie von den auf die Pflanzen oder ihre Umgebung ausgebrachten Wirkstoffmengen und von der Art der Applikation. In jedem Fall sollen Wachstumsregulatoren die Kulturpflanzen in bestimmter gewünschter Weise beeinflussen.

Pflanzenwuchsregulierende Stoffe können zum Beispiel zur Hemmung des vegetativen Wachstums der Pflanzen eingesetzt werden. Eine derartige Wuchshemmung ist unter anderem bei Gräsern von wirtschaftlichem Interesse, denn dadurch kann die Häufigkeit der Grasschnitte in Ziergarten, Park- und Sportanlagen, an Straßenrändern, auf Flughäfen oder in Obstanlagen reduziert werden. Von Bedeutung ist auch die Hemmung des Wuchses von krautigen und holzigen Pflanzen an Straßenrändern und in der Nähe von Pipelines oder Überlandleitungen oder ganz allgemein in Bereichen, in denen ein starker Zuwachs der Pflanzen unerwünscht ist.

Wichtig ist auch die Anwendung von Wachstumsregulatoren zur Hemmung des Längenwachstums von Getreide. Hierdurch wird die Gefahr des Umknickens ("Lagerns") der Pflanzen vor der Ernte verringert oder vollkommen beseitigt. Außerdem können Wachstumsregulatoren bei Getreide eine Halmverstärkung hervorrufen, die ebenfalls dem Lagern entgegenwirkt. Die Anwendung von Wachstumsregulatoren zur Halmverkürzung und Halmverstärkung erlaubt es, hohere Düngermengen auszubringen, um den Ertrag zu steigern, ohne daß die Gefahr besteht, daß das Getreide lagert.

Eine Hemmung des vegetativen Wachstums ermöglicht bei vielen Kulturpflanzen eine dichtere Anpflanzung, so daß Mehrerträge bezogen auf die Bodenfläche erzielt werden können. Ein Vorteil der so erzielten kleineren Pflanzen ist auch, daß die Kultur leichter bearbeitet und beerntet werden kann.

Eine Hemmung des vegetativen Wachstums der Pflanzen kann auch dadurch zu Ertragssteigerungen führen, daß die Nährstoffe und Assimilate in starkerem Maße der Blüten- und Fruchtbildung zugute kommen als den vegetativen Pflanzenteilen.

Mit Wachstumsregulatoren läßt sich haufig auch eine Forderung des vegetativen Wachstums erzielen. Dies ist von groBem Nutzen, wenn die vegetativen Pflanzenteile geerntet werden. Eine Förderung des vegetativen Wachstums kann aber auch gleichzeitig zu einer Forderung des generativen Wachstums führen, dadurch daß mehr Assimilate gebildet werden, so daß mehr oder größere Früchte entstehen.

Ertragssteigerungen können in manchen Fallen durch einen Eingriff in den pflanzlichen Stoffwechsel erreicht werden, ohne daß sich Änderungen des vegetativen Wachstums bemerkbar machen. Ferner kann mit Wachstumsregulatoren eine Veränderung der Zusammensetzung der Pflanzen erreicht werden, was wiederum zu einer Qualitätsverbesserung der Ernteprodukte führen kann. So ist es beispielsweise möglich, den Gehalt an Zucker in Zuckerrüben, Zuckerrohr, Ananas sowie in Zitrusfrüchten zu erhöhen oder den Proteingehalt in Soja oder Getreide zu steigern. Auch ist es beispielsweise möglich, den Abbau erwünschter Inhaltsstoffe, wie z.B. Zucker in Zuckerrüben oder Zuckerrohr, mit Wachstumsregulatoren vor oder nach der Ernte zu hemmen. Außerdem läßt sich die Produktion oder der Abfluß von sekundären Pflanzeninhaltsstoffen positiv beeinflussen. Als Beispiel sei die Stimulierung des Latexflusses bei Gummibäumen genannt.

Unter dem Einfluß von Wachstumsregulatoren kann es zur Ausbildung parthenokarper Früchte kommen. Ferner kann das Geschlecht der Blüten beeinflußt werden. Auch kann eine Sterilitat des Pollens erzeugt werden, was bei der Züchtung und Herstellung von Hybridsaatgut eine große Bedeutung hat.

Durch den Einsatz von Wachstumsregulatoren läßt sich die Verzweigung der Pflanzen steuern. Einerseits kann durch Brechen der Apikaldominanz die Entwicklung von Seitentrieben gefördert werden, was besonders im Zierpflanzenbau auch in Verbindung mit einer Wuchshemmung sehr erwünscht sein kann. Andererseits ist es aber auch möglich, das Wachstum der Seitentriebe zu hemmen. Für diese Wirkung besteht z.B. großes Interesse im Tabakanbau oder bei der Anpflanzung von Tomaten.

Unter dem Einfluß von Wachstumsregulatoren kann der Blattbestand der Pflanzen so gesteuert werden, daß ein Entblättern der Pflanzen zu einem gewünschten Zeitpunkt erreicht wird. Eine derartige Entlaubung spielt bei der mechanischen Beerntung der Baumwolle eine große Rolle, ist aber auch in anderen Kulturen wie z.B. im Weinbau zur Erleichterung der Ernte von Interesse. Eine Entlaubung der Pflanzen kann auch vorgenommen werden, um die Transpiration der Pflanzen vor dem Verpflanzen herabzusetzen.

Ebenso läßt sich mit Wachstumsregulatoren der Fruchtfall steuern. Einerseits kann ein vorzeitiger Fruchtfall verhindert werden. Andererseits kann aber auch der Fruchtfall oder sogar das Abfallen der Blüten bis zu einem gewünschten Maße gefördert werden ("Ausdünnung"), um die Alternanz zu brechen. Unter Alternanz versteht man die Eigenart einiger Obstarten, endogen bedingt von Jahr zu Jahr sehr unterschiedliche Erträge zu bringen. Schließlich ist es möglich, mit Wachstumsregulatoren zum Zeitpunkt der Ernte die zum Ablösen der Früchte erforderlichen Kräfte zu reduzieren, um eine mechanische Beerntung zu ermöglichen oder eine manuelle Beerntung zu erleichtern.

Mit Wachstumsregulatoren läßt sich ferner eine Beschleunigung oder auch Verzögerung der Reife des Erntegutes vor oder nach der Ernte erreichen. Dieses ist von besonderem Vorteil, weil sich dadurch eine optimale Anpassung an die Bedürfnisse des Marktes herbeiführen läßt. Weiterhin können

Wachstumsregulatoren in manchen Fällen die Fruchtausfärbung verbessern. Darüber hinaus kann mit Wachstumsregulatoren auch eine zeitliche Konzentrierung der Reife erzielt werden. Damit werden die Voraussetzungen dafür geschaffen, daß z.B. bei Tabak, Tomaten oder Kaffee eine vollständige mechanische oder manuelle Beerntung in einem Arbeitsgang vorgenommen werden kann.

Durch Anwendung von Wachstumsregulatoren kann ferner die Samen- oder Knospenruhe der Pflanzen beeinflußt werden, so daß die Pflanzen, wie z. B. Ananas oder Zierpflanzen in Gärtnereien, zu einem Zeitpunkt keimen, austreiben oder blühen, an dem sie normalerweise hierzu keine Bereitschaft zeigen. Eine Verzögerung des Austriebes von Knospen oder der Keimung von Samen mit Hilfe von Wachstumsregulatoren kann in frostgefährdeten Gebieten erwünscht sein, um Schädigungen durch Spätfroste zu vermeiden.

Schließlich kann mit Wachstumsregulatoren eine Resistenz der Pflanzen gegen Frost, Trockenheit oder hohen Salzgehalt des Bodens induziert werden. Hierdurch wird die Kultivierung von Pflanzen in Gebieten möglich, die hierzu normalerweise ungeeignet sind.

Die Wirkstoffe können in die üblichen Formulierungen übergeführt werden, wie Lösungen, Emulsionen, Suspensionen, Pulver, Schäume, Pasten, Granulate, Aerosole, Feinstverkapselungen in polymeren Stoffen und in Hüllmassen für Saatgut, sowie ULV-Formulierungen.

Diese Formulierungen werden in bekannter Weise hergestellt, z.B. durch Vermischen der Wirkstoffe mit Streckmitteln, also flüssigen Lösungsmitteln, unter Druck stehenden verflüssigten Gasen und/oder festen Trägerstoffen, gegebenenfalls unter Verwendung von oberflächenaktiven Mitteln, also Emulgiermitteln und/oder Dispergiermitteln und/oder schaumerzeugenden Mitteln. Im Falle der Benutzung von Wasser als Streckmittel können z.B. auch organische Lösungsmittel als Hilfslösungsmittel verwendet werden. Als flüssige Lösungsmittel kommen im wesentlichen in Frage: Aromaten, wie Xylol, Toluol, oder Alkylnaphthaline, chlorierte Aromaten oder chlorierte aliphatische Kohlenwasserstoffe, wie Chlorbenzole, Chlorethylene oder Methylenchlorid, aliphatische Kohlenwasserstoffe, wie Cyclohexan oder Paraffine, z.B. Erdölfraktionen, Alkohole, wie Butanol oder Glycol sowie deren Ether und Ester, Ketone, wie Aceton, Methylethylketon, Methylisobutylketon oder Cyclohexanon, stark polare Lösungsmittel, wie Dimethylformamid und Dimethylsulfoxid, sowie Wasser; mit verflüssigten gasförmigen Streckmitteln oder Tragerstoffen sind solche Flüssigkeiten gemeint, welche bei normaler Temperatur und unter Normaldruck gasförmig sind, z.B. Aerosol-Treibgase, wie Halogenkohlenwasserstoffe sowie Butan, Propan, Stickstoff und Kohlendioxid; als feste Trägerstoffe kommen in Frage: z.B. natürliche Gesteinsmehle, wie Kaoline, Tonerden, Talkum, Kreide, Quarz, Attapulgit, Montmorillonit oder Diatomeenerde und synthetische Gesteinsmehle, wie hochdisperse Kieselsäure, Aluminiumoxid und Silikate; als feste Trägerstoffe für Granulate kommen in Frage: z.B. gebrochene und fraktionierte natürliche Gesteine wie Calcit, Marmor, Bims, Sepiolith, Dolomit sowie synthetische Granulate aus anorganischen und organischen Mehlen sowie Granulate aus organischem Material wie Sägemehl, Kokosnußschalen, Maiskolben und Tabakstengel; als Emulgier und/oder schaumerzeugende Mittel kommen in Frage: z.B. nichtionogene und anionische Emulgatoren, wie Polyoxyethylen-Fettsäure-Ester, Polyoxyethylen-Fettalkohol-Ether, z.B. Alkylarylpolyglykol-Ether, Alkylsulfonate, Alkylsulfate, Arylsulfonate sowie Eiweißhydrolysate; als Dispergiermittel kommen in Frage: z.B. Lignin-Sulfitablaugen und Methylcellulose.

Es können in den Formulierungen Haftmittel wie Carboxymethylcellulose, natürliche und synthetische pulverige, körnige oder latexförmige Polymere verwendet werden, wie Gummiarabicum, Polyvinylalkohol, Polyvinylacetat, sowie natürliche Phospholipide, wie Kephaline und Lecithine, und synthetische Phospholipide. Weitere Additive können mineralische und vegetabile Öle sein.

Es können Farbstoffe wie anorganische Pigmente, z.B. Eisenoxid, Titanoxid, Ferrocyanblau und organische Farbstoffe, wie Alizarin-, Azo- und Metallphthalocyaninfarbstoffe und Spurennährstoffe wie Salze von Eisen, Mangan, Bor, Kupfer, Kobalt, Molybdän und Zink verwendet werden.

Die Formulierungen enthalten im allgemeinen zwischen 0, 1 und 95 Gewichtsprozent Wirkstoff, vorzugsweise zwischen 0,5 und 90 %.

Die erfindungsgemäßen Wirkstoffe können in den Formulierungen in Mischung mit anderen bekannten Wirkstoffen vorliegen wie Fungizide, Insektizide, Akarizide und Herbizide sowie Mischungen mit Düngemitteln und anderen Wachstumsregulatoren.

Die Wirkstoffe können als solche, in Form ihrer Formulierungen oder den daraus bereiteten Anwendungsformen wie gebrauchsfertige Lösungen, emulgierbare Konzentrate, Emulsionen, Schäume, Suspensionen, Spritzpulver, Pasten, lösliche Pulver, Staubemittel und Granulate, angewendet werden. Die Anwendung geschieht in üblicher Weise, z.B. durch Gießen, Verspritzen, Versprühen, Verstreuen, Verstäuben, Verschäumen, Bestreichen usw. Es ist ferner möglich, die Wirkstoffe nach dem Ultra-Low-Volume-Verfahren auszubringen oder die Wirkstoffzubereitung oder den Wirkstoff selbst in den Boden zu injizieren. Es kann auch das Saatgut der Pflanzen behandelt werden.

Beim Einsatz der erfindungsgemäßen Stoffe als Fungizide kann die Aufwandmenge je nach Art der Applikation in einem größeren Bereich variiert werden. So liegen die Wirkstoffkonzentrationen bei der Behandlung von Pflanzenteilen in den Anwendungsformen im allgemeinen zwischen 1 und 0,0001 Gew.-%, vorzugsweise zwischen 0,5 und 0,001 %. Bei der Saatgutbehandlung werden im allgemeinen Wirkstoffmengen von 0,001 bis 50 g je Kilogramm Saatgut, vorzugsweise 0,01 bis 10 g, benotigt. Bei Behandlung des Bodens sind Wirkstoffkonzentrationen von 0,00001 bis 0,1 Gew.-%, vorzugsweise von 0,0001 bis 0,02 %, am Wirkungsort erforderlich.

Beim Einsatz der erfindungsgemäßen Verbindungen als Pflanzenwachstumsregulatoren können die Aufwandmengen in einem größeren Bereich variiert werden. Im allgemeinen verwendet man pro Hektar Bodenfläche 0,01 bis 50 kg, bevorzugt 0,05 bis 10 kg an Wirkstoff.

Beim Einsatz der erfindungsgemäßen Stoffe als Pflanzenwachstumsregulatoren gilt, daß die Anwendung in einem bevorzugten Zeitraum vorgenommen wird, dessen genaue Abgrenzung sich nach den klimatischen und vegetativen Gegebenheiten richtet.

Die Herstellung und die Verwendung der erfindungsgemäßen Wirkstoffe geht aus den nachfolgenden Beispielen hervor.

<u>Herstellungsbeispiele</u>

<u>Beispiel 1</u>

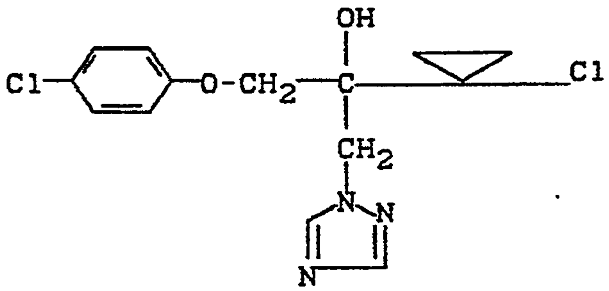

(I-1)

In ein Gemisch aus 29 g (0,42 Mol) 1,2,4-Triazol, 19 g (0,14 Mol) Kaliumcarbonat und 100 ml Acetonitril wird unter Stickstoffatmosphäre eine Lösung 27,9 g (0,11 Mol) 2-(1-Chlorcyclopropyl)-2-(4-chlor-phenoxymethyl)-oxiran in 50 ml Acetonitril eingetropft, wobei das Reaktionsgemisch unter Rückfluß gekocht wird. Nach beendeter Zugabe wird noch weitere 8 Stunden unter Rückfluß erhitzt. Anschließend saugt man vom festen Rückstand ab und engt das Filtrat unter vermindertem Druck ein. Der verbleibende Rückstand wird in Essigester aufgenommen. Die dabei entstehende Lösung wird mit Wasser gewaschen, über Natriumsulfat getrocknet und unter vermindertem Druck eingeengt, Das so erhaltene Produkt wird auf chromatographischem Wege über Kieselgel mit Chloroform/Ethanol = 97:3 als Laufmittel gereinigt. Man erhalt auf diese Weise 13,7 g (39% der Theorie) an 2-(1-Chlorcyclopropyl)-1-(4-chlorphenoxy)-3-(1,2 4-triazol-1-yl)propan-2-ol in Form eine Öles.

$^1$H-NMR (80 MHz, $CDCl_3$):
δ = 0,5–1, 4(m,4H), 4, 1–4,4(m, 3H),
4,75 (s, 2H), 6, 8–7, 25 (m, 4H),
8,0 (s, 1H), 8,17 (s, 1H)

<u>Herstellung von Ausgangsprodukten</u>

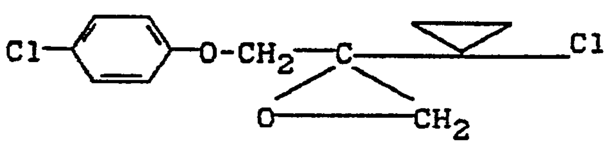

(II-1)

In ein Gemisch aus 3,9 g (0,13 Mol) Natriumhydrid (80%ig) und 27 g (0,12 Mol) Trimethyl-oxosulfoniumiodid werden bei 10°C unter Stickstoffatmosphare und unter Rühren 90 ml absolutes Dimethylsulfoxid zugetropft. Man rührt noch 1 Stunde bei Raumtemperatur, kühlt dann auf 10°C ab und tropft eine Lösung von 27 g (0,11 Mol) 1-Chlorcyclopropyl-4-chlorphenoxymethyl-keton in 40 ml absolutem Dimethylsulfoxid hinzu. Das Reaktionsgemisch wird zunächst 48 Stunden bei 20°C gerührt, dann 1 Stunde auf 40°C erwärmt und danach auf Wasser gegossen. Das entstehende Gemisch wird mit Essigester extrahiert. Die organische Phase wird mit Wasser gewaschen, über Natriumsulfat getrocknet und unter vermindertem Druck eingeengt. Man erhält auf diese Weise 27,9 g (98% der Theorie) an 2-(1-Chlorcyclopropyl)-2-(4-chlorphenoxymethyl)-oxiran in Form eines Öles.

$^1$H-NMR (60 MHz, $CDCl_3$):
δ= 0, 75 – 1,4 (m, 4H), 2,8 (d, 1H), 3,05 (d, 1H),
4,25 (d, 1H), 4,58 (d, 1H), 6,8 – 7,4 (m, 4H).

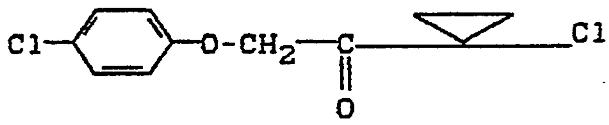

(VII-1)

In eine Lösung von 51 g (0,33 Mol) 1-Chlor-1-chloracetyl-cyclopropan in 250 ml Acetonitril werden nacheinander 49 g (0,38 Mol) 4-Chlorphenol und 70 g (0,51 Mol) Kaliumcarbonat gegeben. Das Gemisch wird 8 Stunden unter Rückfluß erhitzt, dann filtriert und eingeengt durch Abziehen des Lösungsmittels. Man nimmt den Rückstand in Essigester auf, wäscht nacheinander mit verdünnter, wäßriger Natronlauge und Wasser, trocknet über Natriumsulfat und zieht unter vermindertem Druck das Lösungsmittel ab. Der Rückstand wird einer Destillation unterworfen. Man erhält auf diese Weise 27 g (33% der Theorie) an 1-Chlor-cyclopropyl-4-chlorphenoxy-methylketon in Form einer Flüssigkeit vom Siedepunkt 125 –127°C/0,1 mbar.

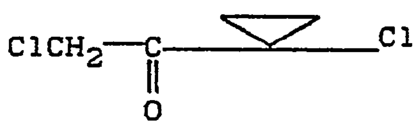

(X-1)

Bei Raumtemperatur werden 40,5 ml (0,5 Mol) Sulfurylchlorid unter Rühren langsam in eine Lösung von 54 g (0 46 Mol) 1-Acetyl-1-chlor-cyclopropan in 250 ml Methylenchlorid eingetropft. Es wird zunächst 14 Stunden bei Raumtemperatur und dann 30 Minuten bei 30°C gerührt. Das Reaktionsgemisch wird dann nacheinander mit gesättigter, wäßriger Natriumhydrogencarbonat-Lösung und mit Wasser gewaschen, Danach wird die organische Phase über Natriumsulfat getrocknet und unter vermindertem Druck eingeengt. Man erhält auf diese Weise 51,5 g (74% der Theorie) an 1-Chlor-1-chloracetylcyclopropan in Form einer öligen Substanz.

$^1$H-NMR (60 MHz, $CDCl_3$):
δ= 1,2 – 1,9 (m, 4H); 4,8 (s, 2H)

Beispiel 2

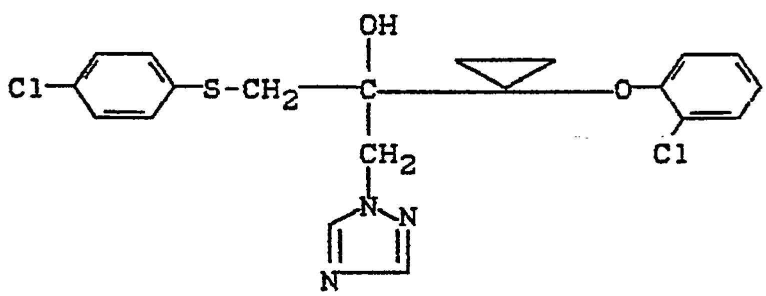

(I-2)

Ein Gemisch aus 15 g (0,05 Mol) 2-[1-(2-Chlorphenoxy-cyclopropyl]-2-[(1,2,4-triazol-1-yl)-methyl]-oxiran, 7,5 g (0,05 Mol) 4-Chlor-thiophenol, 0,75 g (0,13 Mol) Kaliumhydroxid-Pulver und 45 ml Acetonitril wird unter Rückfluß erhitzt. Man filtriert das Reaktionsgemisch und engt das Filtrat durch Abziehen des Lösungsmittels unter vermindertem Druck ein. Das dabei verbleibende Produkt wird einer chromatographischen Reinigung unterworfen. Man erhält auf diese Weise 22 g (98% der Theorie) an 1-(4-Chlorphenylmercapto)-2-[1-(2-chlorphenoxy)-cyclopropyl-3-(1,2,4-triazol-1-yl)-propan-2-ol in Form eines öligen Produktes.

$^1$H-NMR (80 MHz, $CDCl_3$):
δ= 0,3-1,3 (m, 4H), 3,35 (d, 1H), 3,58 (d, 1H), 4,30 (s, 1H),
4,77 (s, 2H), 7,0–7,5 (m, 8H),
8,08 (s, 1H), 8,5 (s, 1H).

Herstellung von Ausgangssubstanzen:

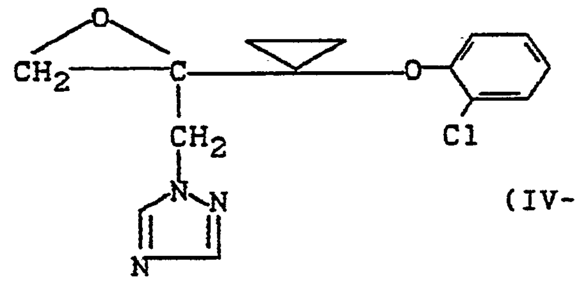

(IV-1)

In ein Gemisch aus 6 g (0,2 Mol) Natriumhydrid (80%ig) und 42 g (0,19 Mol) Trimethyloxosulfonium-iodid werden bei 10°C unter Stickstoffatmosphäre und unter Rühren 135 ml absolutes Dimethylsulfoxid eingetropft. Das Reaktionsgemisch wird 1 Stunde bei Raumtemperatur gerührt, dann auf 10°C abgekühlt und tropfenweise mit einer Lösung von 47 g (0,17 Mol) 1-(2-Chlorphenoxy-cyclopropyl-(1,2,4-triazol-1-yl)-methyl-keton in 50 ml absolutem Dimethylsulfoxid versetzt. Man rührt weitere 48 Stunden bei Raumtemperatur und erwärmt dann 1 Stunde auf 40°C. Man gießt danach das Reaktionsgemisch auf Wasser und extrahiert mit Essigester. Die vereinigten organischen Phasen werden mit Wasser gewaschen und nach dem Trocknen über Natriumsulfat durch Abziehen des Lösungsmittels unter vermindertem Druck eingeengt. Man erhält auf diese Weise 40,2 g (81% der Theorie) an 2-[1-(2-Chlorphenoxy)-cyclopropyl]-2-[(1,2,4-triazol-1-yl-methyl]oxiran in Form eines öligen Produktes.

$^1$H-NMR (60 MHz, $CDCl_3$):
δ= 0,6 – 1,6 (m, 4H), 2,6 (d, 1H), 2,78 (d, 1H),
4,34 (d, 1H), 4,75 (d, 1H), 6,9 – 7,5 (m, 4H),
8,0 (s, 1H), 8,45 (s, 1H).

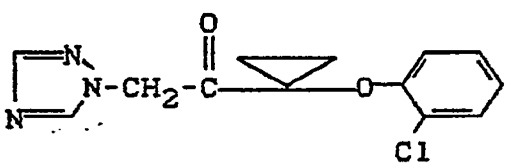

(XII-1)

In ein Gemisch aus 38,5 g (0,28 Mol) Kaliumcarbonat, 58,4 g (0,85 Mol) 1,2,4-Triazol und 140 ml Acetonitril wird eine Lösung von 62 g (0,25 Mol) 1-Chloracetyl-1-(2-chlorphenoxy)-cyclopropan in 60 ml Acetonitril eingetropft, wobei das Reaktionsgemisch unter Rückfluß gekocht wird. Das Reaktionsgemisch wird weitere 8 Stunden unter Rückfluß erhitzt, dann filtriert und durch Abziehen des Lösungsmittels unter vermindertem Druck eingeengt, Man nimmt den verbleibenden Rückstand in Essigester auf, wäscht mit Wasser, trocknet über Natriumsulfat und zieht dann das Lösungsmittel unter vermindertem Druck ab. Das verbleibende Produkt wird mit Chloroform/Ethanol = 99:1 als Laufmittel an Kieselgel chromatographiert, Man erhält auf diese Weise 47 g (67% der Theorie) an 1-(2-Chlorphenoxy)-cyclopropyl-(1,2, 4 triazol-1-yl)-methylketon in Form eines öligen Produktes.
$^1$H-NMR (60 MHz, $CDCl_3$):
= 1,15–1,85 (m, 4H), 5,34 (s, 2H),
6 80–7,60 (m, 4H), 7,90 (s, 1H),
8,07 (s, 1H).

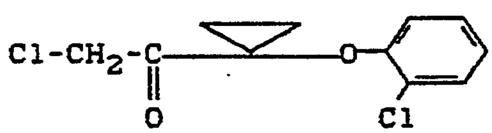

(X-2)

In ein Gemisch aus 61,7 g (0,29 Mol) 1-Acetyl-1-(2-chlorphenoxy)-cyclopropan und 0,4 g Dibenzoylperoxid werden bei 20°C unter Rühren und unter Kühlung 48 g (0,35 Mol) Sulfurylchlorid eingetropft. Man rührt weitere 4 Stunden bei 20°C und belichtet das Reaktionsgemisch gleichzeitig mit einer 200 W-Glühbirne. Anschließend wird das Reaktionsgemisch mit einem Gemisch aus Essigester und Toluol versetzt. Es wird mit Wasser gewaschen, über Natriumsulfat getrocknet und durch Abziehen des Lösungsmittels unter vermindertem Druck eingeengt. Das verbleibende Produkt wird einer Vakuumdestillation

unterworfen, Man erhält auf diese Weise 62,3 g (87% der Theorie) an 1-Chloracetyl-1-(2-chlorphenoxy)-cyclopropan.

Kp = 117–122°C/0,2 mbar

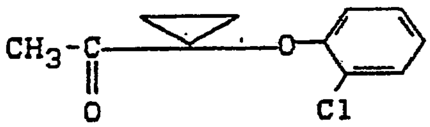

(XI-1)

In ein Gemisch aus 100 g (0,83 Mol) 5-Chlorpentan-2-on und 400 ml Methylenchlorid wird bei 10°C unter Rühren eine Lösung von 134 g (0,84 Mol) Brom in 130 ml Methylenchlorid eingetropft. Nach beendeter Zugabe wird das Reaktionsgemisch noch 15 Minuten nachgerührt und dann auf 500 ml Wasser gegossen. Die organische Phase wird abgetrennt, nacheinander mit 5%iger wäßriger Natriumcarbonat-Lösung und mit Wasser gewaschen, dann über Natriumsulfat getrocknet und durch Abziehen des Lösungsmittels unter vermindertem Druck eingeengt. Der Rückstand wird in 200 ml Dimethylformamid aufgenommen, und die entstehende Lösung wird bei 60°C in ein Gemisch aus 230 g Kaliumcarbonat, 106 g (0,83 Mol) 2-Chlorphenol und 400 ml Dimethylformamid eingetropft. Das Reaktionsgemisch wird zunächst 4 Stunden bei 60°C und dann 4 Stunden bei 90°C gerührt. Danach saugt man vom festen Rückstand ab, zieht das Lösungsmittel unter vermindertem Druck ab und nimmt den verbleibenden Rückstand in einem Gemisch aus Essigester und Toluol auf. Die organische Phase wird mit Wasser gewaschen, über Natriumsulfat getrocknet und durch Abziehen des Losungsmittels unter vermindertem Druck eingeengt. Der verbleibende Rückstand wird einer Vakuumdestillation unterworfen. Man erhalt auf diese Weise 92,5 g (53% der Theorie) an 1-Acetyl-l(2-chlorphenoxy)-cyclopropan in Form einer Flüssigkeit vom Siedepunkt 81°C / 0, 2 mbar.

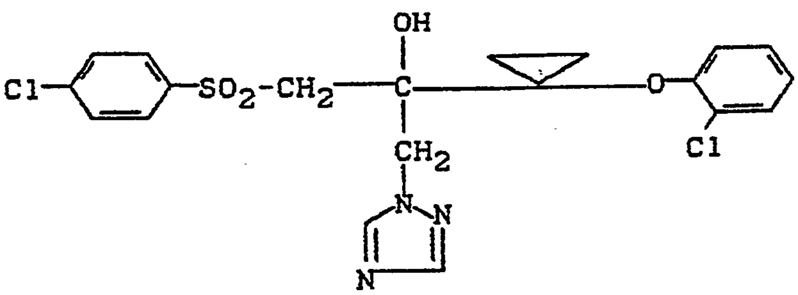

(I-3)

Eine Lösung von 10 g (23 mMol) 1-(4-Chlorphenylmercapto)-2-[1-(2-chlorphenoxy)-cyclopropyl]-3-(1,2,4triazol-1-yl)-propan-2-ol in 50 ml Eisessig wird auf 80°C erwärmt und unter Rühren tropfenweise mit 20 ml 35%iger Wasserstoffperoxid-Lösung versetzt. Das Reaktionsgemisch wird noch 4 Stunden auf 90°C erwärmt und dann auf Wasser gegossen. Man saugt das feste Produkt ab, nimmt es in Methylenchlorid auf, wäscht nacheinander mit verdünnter, wäßriger Natronlauge und Wasser, trocknet über Natriumsulfat und engt durch Abziehen des Lösungsmittels unter vermindertem Druck ein. Man erhält auf diese Weise 6,4 g (60% der Theorie) an 1-(4-Chlorphenylsulfonyl)-2-[1-(2-chlorphenoxy)-cyclopropyl]-3-(1,2,4-triazol-1-yl)-propan-2-ol.

$^1$H-NMR (200 MHz, $CDCl_3$):
δ= 0,30 – 0,95 (m, 4H), 3,65 (d, 1H), 3,87 (d, 1H),
4,85 (d, 1H), 5,11 (d, 1H), 7,0–7,4 (m, 4H),
7,55 (d, 2H), 7,80 (d, 2H),
8,0 (s, IH), 8,44 (s, IH).

Nach den in den Beispielen 1 bis 3 und den in der Beschreibung angegebenen Methoden werden auch die in der folgenden Tabelle 2 formelmäßig aufgeführten Stoffe hergestellt.

Tabelle 2

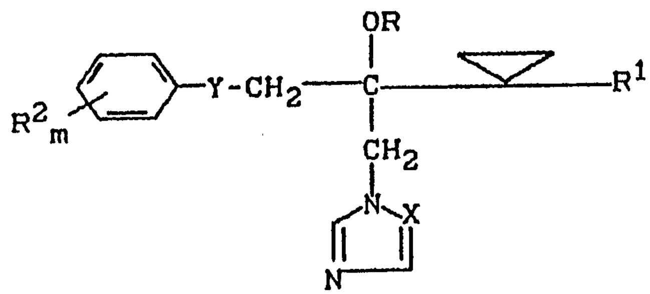

| Bsp. Nr. | Verbindung Nr. | $R^2_m$ | R | $R^1$ | Y | X | Schmelz-punkt (°C) |
|---|---|---|---|---|---|---|---|
| 4 | I-4 | 4-F | H | Cl | O | N | *) |
| 5 | I-5 | 2,4-$Cl_2$ | H | 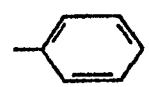 | O | N | 123 |
| 6 | I-6 | 2-$CH_3$,4-Cl | H | 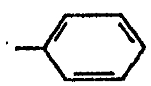 | O | N | 125 |
| 7 | I-7 | 4-CH=N-$OCH_3$ | H | 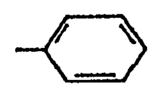 | O | N | 88 |
| 8 | I-8 | 4-Cl | H | 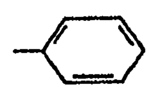 | O | N | 140 |
| 9 | I-9 | 4-Cl | H | 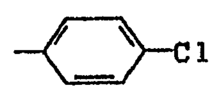 | O | N | Harz |

<u>Tabelle 2</u> (Fortsetzung)

| Bsp. Nr. | Verbindung Nr. | $R^2_m$ | R | $R^1$ | Y | X | Schmelzpunkt (°C) |
|---|---|---|---|---|---|---|---|
| 10 | I-10 | 2,4-$Cl_2$ | H | 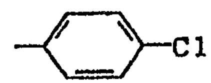 | O | N | Harz |
| 11 | I-11 | 4-$OCF_3$ | H | 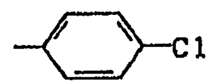 | O | N | Harz |
| 12 | I-12 | 2-$CH_3$,4-Cl | H | 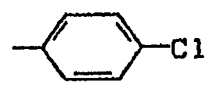 | O | N | 140 |
| 13 | I-13 | 4-CH=N-$OCH_3$ | H | 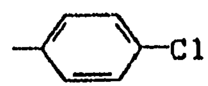 | O | N | 113 |
| 14 | I-14 | 4-F | H | 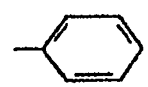 | O | N | 142 |
| 15 | I-15 | $CF_3$ | H | 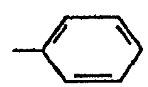 | O | N | 158 |
| 16 | I-16 | 4-$CH_3$ | H | 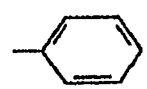 | O | N | 137 |
| 17 | I-17 | 4-$OCF_3$ | H | 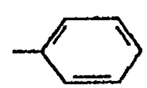 | O | N | 148 |

Tabelle 2 (Fortsetzung)

| Bsp. Nr. | Verbindung Nr. | $R^2_m$ | R | $R^1$ | Y | X | Schmelzpunkt (°C) |
|---|---|---|---|---|---|---|---|
| 18 | I-18 | $4-CF_3$ | H | 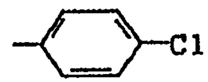 | O | N | 127 |
| 19 | I-19 | $4-CH_3$ | H | 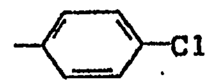 | O | N | Harz |
| 20 | I-20 | 2-Cl | H | 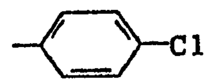 | O | N | Harz |
| 21 | I-21 | 4-F | H | 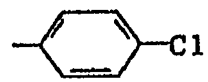 | O | N | 124 |
| 22 | I-22 | 2-Cl | H | 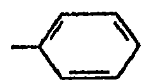 | O | N | 136 |
| 23 | I-23 | $2-CH_3$,4-Cl | H | 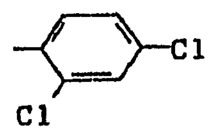 | O | N | Harz |
| 24 | I-24 | 4-Cl | H | 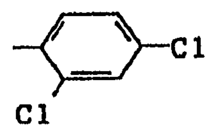 | O | N | 118 |
| 25 | I-25 | $4-CH=N-OCH_3$ | H | 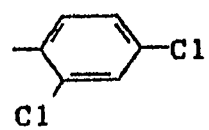 | O | N | Harz |

Tabelle 2 (Fortsetzung)

| Bsp. Nr. | Verbindung Nr. | $R^2_m$ | R | $R^1$ | Y | X | Schmelzpunkt (°C) |
|---|---|---|---|---|---|---|---|
| 26 | I-26 | 2,4-$Cl_2$ | H | 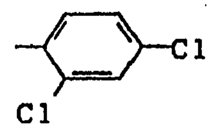 | O | N | Harz |
| 27 | I-27 | 2-Cl,4-$OCF_3$ | H | 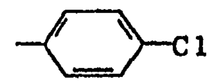 | O | N | 113 |
| 28 | I-28 | 4-$OCF_3$ | H | 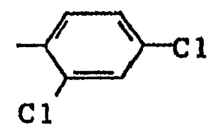 | O | N | 85 |
| 29 | I-29 | 4-Cl | H | 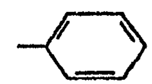 | S | N | 108 |

*) Die im Beispiel 4 offenbarte Verbindung ist durch das $^1H$-NMR-Spektrum (360 MHz, $CDCl_3$ charakterisiert:

δ = 0,55 - 0,70 (m, 2H), 0,80 - 0,95 (m, 1H), 1,10 - 1,20 (m, 1H) 4,10 (s, 1H), 4,16 (d, 1H), 4,30 (d, 1H), 4,75 (d, 1H), 4,80 (d, 1H), 6,80 - 7,05 (m, 4H), 8,02 (s, 1H), 8,30 (s 1H).

In den folgenden Verwendungsbeispielen wurde die Verbindung der nachstehend angegebenen Formel als Vergleichssubstanz eingesetzt:

(A) = 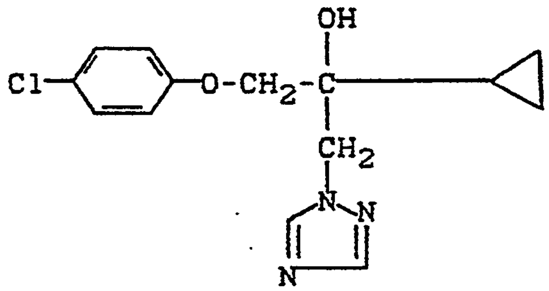

(Bekannt aus EP-A 0 040 345).

Beispiel A

Sphaerotheca-Test (Gurke) / protektiv
Lösungsmittel: 4,7 Gewichtsteile Aceton
Emulgator: 0,3 Gewichtsteile Alkyl-aryl-polyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit bespritzt man junge Pflanzen mit der Wirkstoffzubereitung bis zur Tropfnässe. Nach Antrocknen des Spritzbelages werden die Pflanzen mit Konidien des Pilzes Sphaerotheca fuliginea bestäubt.

Die Pflanzen werden anschließend bei 23 bis 24°C und bei einer relativen Luftfeuchtigkeit von ca. 75 % im Gewächshaus aufgestellt.

10 Tage nach der Inokulation erfolgt die Auswertung.

In diesem Test zeigt die erfindungsgemäße Verbindung der Formel (I-1) eine deutlich bessere Wirkung als die Vergleichssubstanz (A).

Beispiel B

Leptosphaeria nodorum-Test (Weizen) / protektiv
Lösungsmittel: 100 Gewichtsteile Dimethylformamid
Emulgator: 0,25 Gewichtsteile Alkylarylpolyglykolether

Zur Herstellung einer zweckmäßigen Wirkstoffzubereitung vermischt man 1 Gewichtsteil Wirkstoff mit den angegebenen Mengen Lösungsmittel und Emulgator und verdünnt das Konzentrat mit Wasser auf die gewünschte Konzentration.

Zur Prüfung auf protektive Wirksamkeit besprüht man junge Pflanzen mit der Wirkstoffzubereitung taufeucht. Nach Antrocknen des Spritzbelages werden die Pflanzen mit einer Sporensuspension von Leptosphaeria nodorum besprüht. Die Pflanzen verbleiben 48 Stunden bei 20°C und 100% relativer Luftfeuchtigkeit in einer Inkubationskabine.

Die Pflanzen werden in einem Gewächshaus bei einer Temperatur von ca. 15°C und einer relativen Luftfeuchtigkeit von ca. 80% aufgestellt.

10 Tage nach der Inokulation erfolgt die Auswertung. Dabei bedeutet 0% ein Wirkungsgrad, der demjenigen der unbehandelten Kontrolle entspricht, wahrend ein Wirkungsgrad von 100% bedeutet, daß kein Befall vorhanden ist.

In diesem Test zeigt die erfindungsgemäße Verbindung (I-1) eine deutlich bessere Wirksamkeit als die Vergleichssubstanz (A).

Beispiel C

Wachstum bei Sojabohnen
Lösungsmittel: 30 Gewichtsteile Dimethylformamid
Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Sojabohnenpflanzen werden im Gewächshaus bis zur vollen Entfaltung des ersten Folgeblattes angezogen. In diesem Stadium werden die Pflanzen tropfnaß mit den Wirkstoffzubereitungen besprüht. Nach zwei Wochen wird bei allen Pflanzen der Zuwachs gemessen und das Wachstum in Prozent des Zuwachses der Kontrollpflanzen berechnet. Es bedeuten 100% Wachstum ein Wachstum entsprechend dem der Kontrollpflanzen und 0% den Stillstand des Wachstums. Werte über 100% kennzeichnen eine Wuchsförderung, während Werte unter 100% eine Wuchshemmung anzeigen.

In diesem Test zeigt die erfindungsgemäße Verbindung (I-1) eine sehr gute wuchshemmende Wirkung.

Beispiel D

Wachstum bei Baumwollelösungsmittel: 30 Gewichtsteile Dimethylformamid

Emulgator: 1 Gewichtsteil Polyoxyethylen-Sorbitan-Monolaurat

Zur Herstellung einer zweckmäßigen Wirkstoffzubereitung vermischt man 1 Gewichtsteil Wirkstoff mit den angegebenen Mengen Lösungsmittel und Emulgator und füllt mit Wasser auf die gewünschte Konzentration auf.

Baumwollpflanzen werden im Gewächshaus bis zur vollen Entfaltung des fünften Folgeblattes angezogen. In diesem Stadium werden die Pflanzen tropfnaß mit den Wirkstoffzubereitungen besprüht. Nach zwei Wochen wird bei allen Pflanzen der Zuwachs gemessen und das Wachstum in Prozent des Zuwachses der Kontrollpflanzen berechnet. Es bedeuten 100% Wachstum ein Wachstum entsprechend dem der Kontrollpflanzen und 0% den Stillstand des Wachstums. Werte über 100% kennzeichnen eine Wuchsförderung, während Werte unter 100% eine Wuchshemmung anzeigen.

In diesem Test zeigt die erfindungsgemäße Verbindung (I-1) eine sehr gute wuchshemmende Wirkung.

**Patentansprüche**

1. Hydroxyethyl-azolyl-Derivate der Formel

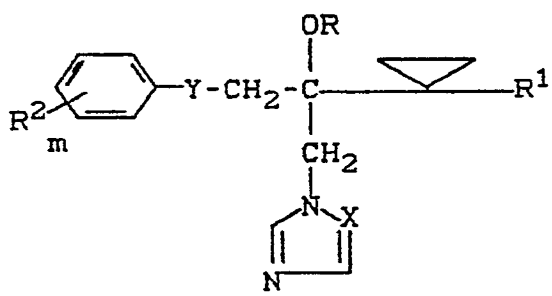

(I)

in welcher

R für Wasserstoff, Alkyl mit 1 bis 6 Kohlenstoffatomen oder Alkylcarbonyl mit 1 bis 6 Kohlenstoffatomen in der Alkylgruppe steht,

$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung $-Z-R^3$ steht, worin

Z für Sauerstoff, Schwefel, SO oder $SO_2$ steht und

$R^3$ für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht,

$R^2$ für Fluor, Chlor, Brom, Methyl, Ethyl, Isopropyl, tert.-Butyl, Methoxy, Methylthio, Trifluormethyl, Trifluormethoxy, Trifluormethylthio, Methoximinomethyl, gegebenenfalls durch Fluor, Chlor und/oder Methyl substituiertes Phenyl oder für gegebenenfalls durch Fluor, Chlor und/oder Methyl substituiertes Phenoxy steht,

m für die Zahlen 0, 1, 2 oder 3 steht,

X für Stickstoff oder eine CH-Gruppe steht und

Y für Sauerstoff, Schwefel, SO oder $SO_2$ steht

sowie deren Säureadditions-Salze und Metallsalz-Komplexe.

2. Verfahren zur Herstellung von Hydroxyethyl-azolyl-Derivaten der Formel (I) gemäss Anspruch 1 sowie von deren Säureadditions-Salzen und Metallsalz-Komplexen, dadurch gekennzeichnet, daß man

a) Oxirane der Formel

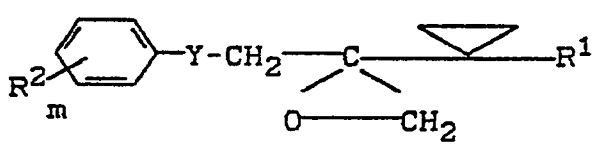

(II)

in welcher

$R^1$, $R^2$, Y und m die oben angegebene Bedeutung haben, mit Azolen der Formel

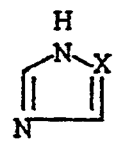

(III)

in welcher
X die oben angegebene Bedeutung hat, in Gegenwart eines Säurebindemittels und in Gegenwart eines Verdünnungsmittels umsetzt,
oder
b) Azolyl-Derivate der Formel

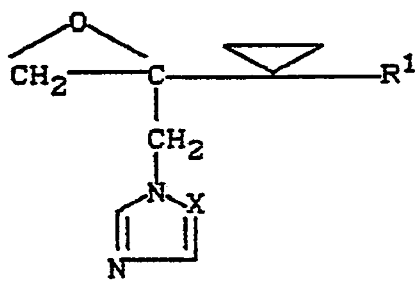

(IV)

in welcher
$R^1$ und X die oben angegebene Bedeutung haben, mit Phenyl-Derivaten der Formel

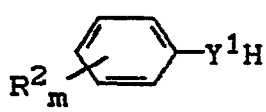

(V)

in welcher
$R^2$ und m die oben angegebene Bedeutung haben und $Y^1$ für Sauerstoff oder Schwefel steht, in Gegenwart eines Säurebindemittels und in Gegenwart eines Verdünnungsmittels umsetzt,
oder
c) Hydroxyethyl-azolyl-Derivate der Formel

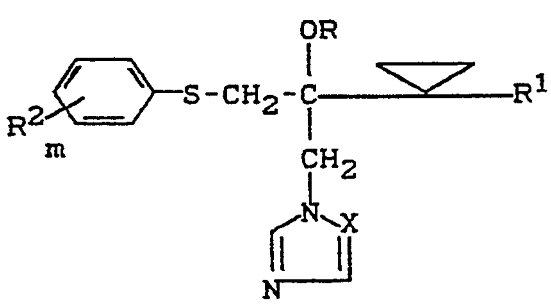

(Ia)

in welcher
R, $R^1$, $R^2$, X und m die oben angegebene Bedeutung haben mit Oxidationsmitteln gegebenenfalls in Gegenwart eines Verdünnungsmittels umsetzt
oder
d) Hydroxyethyl-azolyl-Derivate der Formel

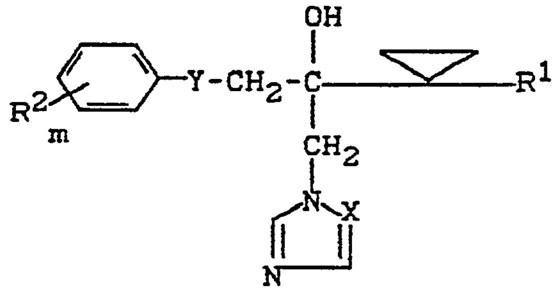

(Ib)

in welcher
$R^1$, $R^2$, X, Y und m die oben angegebene Bedeutung haben, mit starken Basen in Gegenwart eines Verdünnungsmittels umsetzt und die dabei entstehenden Alkoholate der Formel

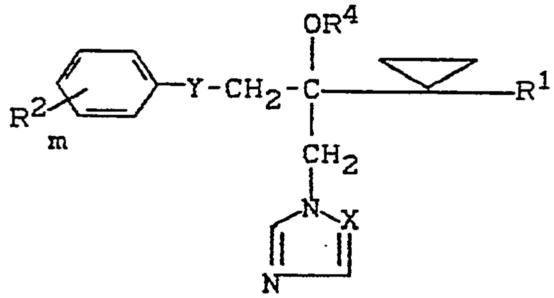

(Ic)

in welcher
$R^1$, $R^2$, X, Y und m die oben angegebene Bedeutung haben, und
$R^4$ für ein Alkalimetallkation, oder für ein quarternäres Ammonium oder Phosphonium-kation steht, mit Halogenverbindungen der Formel

$R^5$ - Hal (VI)

in welcher
$R^5$ für Alkyl mit 1 bis 6 Kohlenstoffatomen oder Alkylcarbonyl mit 1 bis 6 Kohlenstoffatmen in der Alkylgruppe steht
und Hal für Chlor oder Brom steht,
in Gegenwart eines Verdünnungsmittels umsetzt,
und gegebenenfalls anschließend an die so erhaltenen Verbindungen der Formel (I) eine Säure oder ein Metallsalz addiert.

3. Fungizide und pflanzenwuchsregulierende Mittel, gekennzeichnet durch einen Gehalt an mindestens einem Hydroxyethyl-azolyl-Derivat der Formel (I) gemäß Anspruch 1 bzw. an einem Säureadditions-Salz oder Metallsalz-Komplex eines Hydroxyethyl-azolyl-Derivates der Formel (I).

4. Verwendung von Hydroxyethyl-azolyl-Derivaten der Formel (I) gemäß Anspruch 1 bzw. von deren Säureadditions-Salzen und Metallsalz-Komplexen zur Bekämpfung von Pilzen sowie zur Regulierung des Pflanzenwachstums.

5. Oxirane der Formel

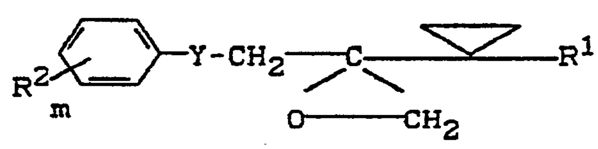

(II)

in welcher
$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung $-Z-R^3$ steht, worin
Z für Sauerstoff, Schwefel, SO oder $SO_2$ steht und
$R^3$ für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht,

$R^2$ für Fluor, Chlor, Brom, Methyl, Ethyl, Isopropyl, tert.-Butyl, Methoxy, Methylthio, Trifluormethyl, Trifluormethoxy, Trifluormethylthio, Methoximinomethyl, gegebenenfalls durch Fluor, Chlor und/oder Methyl substituiertes Phenyl oder für gegebenenfalls durch Fluor, Chlor und/oder Methyl substituiertes Phenoxy steht,
m für die Zahlen 0, 1, 2 oder 3 steht und
Y für Sauerstoff, Schwefel, SO oder $SO_2$ steht.

6. Verfahren zur Herstellung von Oxiranen der Formel (II) gemäß Anspruch 5, dadurch gekennzeichnet, daß man
e) Cyclopropylketone der Formel

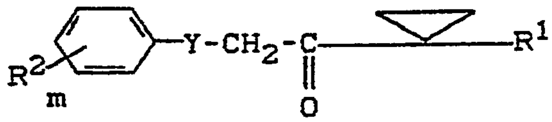

(VII)

in welcher
$R^1$, $R^2$, Y und m die oben angegebene Bedeutung haben,
entweder
α) mit Dimethyloxosulfonium-methylid der Formel

$$\overset{\delta\ominus\,\delta\ominus}{(CH_3)_2SOCH_2} \qquad (VIII)$$

oder
β) mit Dimethylsulfonium-methylid der Formel

$$(CH_3)_2\overset{\delta\ominus}{S}\;\;\overset{\delta\ominus}{CH_2} \qquad (IX)$$

in Gegenwart eines Verdünnungsmittels umsetzt.

7. Cyclopropylketone der Formel

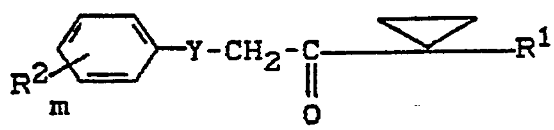

(VII)

in welcher
$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung $-Z-R^3$
Z für Sauerstoff, Schwefel, SO oder $SO_2$ steht und
$R^3$ für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht,
$R^2$ für Fluor, Chlor, Brom, Methyl, Ethyl, Isopropyl, tert.-Buthyl, Methoxy, Methylthio, Trifluormethyl, Trifluormethoxy, Trifluormethylthio, Methoximinomethyl, gegebenenfalls durch Fluor, Chlor und/oder Methyl substituiertes Phenyl oder für gegebenenfalls durch Fluor, Chlor und/oder Methyl substituiertes Phenoxy steht,
m für die Zahlen 0, 1, 2 oder 3 steht und
Y für Sauerstoff, Schwefel, SO oder $SO_2$ steht.

8. Verfahren zur Herstellung von Cyclopropylketonen der Formel (VII) gemäß Anspruch 7, dadurch gekennzeichnet, daß man
f) Halogenketone der Formel

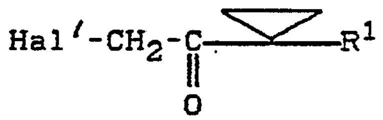 (X)

in welcher
$R^1$ die oben angegebene Bedeutung hat und Hal' für Chlor oder Brom steht, mit Phenyl-Derivaten der Formel

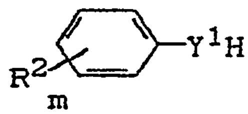 (V)

in welcher
$R^2$ und m die oben angegebene Bedeutung haben und $Y^1$ für Sauerstoff oder Schwefel steht, in Gegenwart eines Säurebindemittels und gegebenenfalls in Gegenwart eines Verdünnungsmittels umsetzt und gegebenenfalls anschließend oxidiert.

9. Azolyl-Derivate der Formel

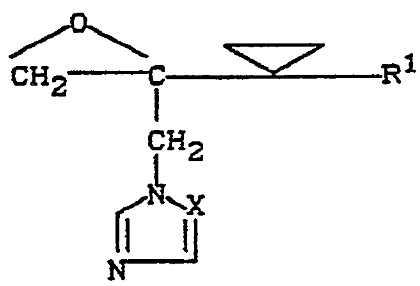 (IV)

in welcher
$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung -Z-$R^3$ steht, worin
Z für Sauerstoff, Schwefel, SO oder $SO_2$ steht und
$R^3$ für gegebenenfalls durch Halogen, Halogenalkyl mit 1 oder 2 Kohlenstoffatomen
und 1 bis 5 Halogenatomen, Alkyl mit 1 bis 4 Kohlenstoffatomen und/oder Alkoxy mit 1 bis 4 Kohlenstoffatomen substituiertes Phenyl steht,
und
X für Stickstoff oder eine CH-Gruppe steht.

10. Verfahren zur Herstellung von Azolyl-Derivaten der Formel (IV) gemäss Anspruch 9, dadurch gekennzeichnet, dass man gekennzeichnet, daß man
h) Azolylketone der Formel

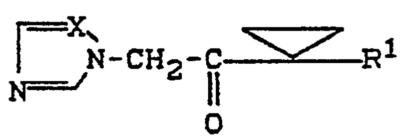 (XII)

in welcher
$R^1$ und X die oben angegebene Bedeutung haben,
entweder
α) mit Dimethyloxosulfonium-methylid der Formel

$$\overset{\delta\ominus}{(CH_3)_2SO}\ \overset{\delta\ominus}{CH_2} \qquad (VIII)$$

oder

β) mit Dimethylsulfonium-methylid der Formel

$$\overset{\delta\oplus}{(CH_3)_2S}\quad\overset{\delta\ominus}{CH_2} \qquad (IX)$$

in Gegenwart eines Verdünnungsmittels umsetzt.

11. Azolylketone der Formel

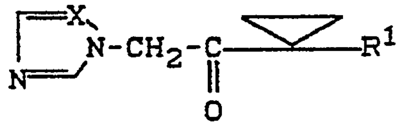

(XII)

in welcher

$R^1$ für Fluor, Chlor, Brom, für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl oder für die Gruppierung -Z-$R^3$ steht, worin

Z für Sauerstoff, Schwefel, SO oder $SO_2$ steht und

$R^3$ für gegebenenfalls durch Halogen, Halogenalkyl mit 1 oder 2 Kohlenstoffatomen und 1 bis 5 Halogenatomen, Alkyl mit 1 bis 4 Kohlenstoffatomen und/oder Alkoxy mit 1 bis 4 Kohlenstoffatomen substituiertes Phenyl steht,

und

X für Stickstoff oder eine CH-Gruppe steht.

12. Verfahren zur Herstellung von Azolylketonen der Formel (XII) gemäß Anspruch 11, durch gekennzeichnet, daß man

i) Halogenkeotne der Formel

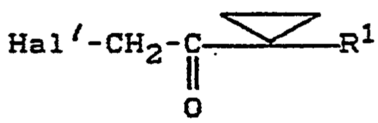

(X)

in welcher $R^1$ die oben angegebene Bedeutung hat und Hal' für Chlor oder Brom steht,

mit Azolen der Formel

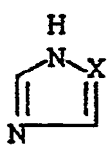

(III)

in welcher

X die oben angegebene Bedeutung hat,

in Gegenwart eines Säurebindemittels und in Gegenwart eines Verdünnungsmittels umsetzen.

## Claims

1. Hydroxyethyl-azolyl derivatives of the formula

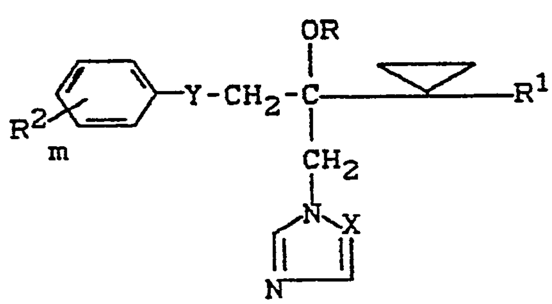

(I)

in which

R represents hydrogen, alkyl having 1 to 6 carbon atoms or alkylcarbonyl having 1 to 6 carbon atoms in the alkyl group,

$R^1$ represents fluorine, chlorine, bromine, phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or represents the grouping $-Z-R^3$,

wherein

Z represents oxygen, sulphur, SO or $SO_2$ and

$R^3$ represents phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms,

$R^2$ represents fluorine, chlorine, bromine, methyl, ethyl, isopropyl, tert.-butyl, methoxy, methylthio, trifluoromethyl, trifluoromethoxy, trifluoromethylthio, methoximinomethyl, phenyl which is optionally substituted by fluorine, chlorine and/or methyl, or represents phenoxy which is optionally substituted by fluorine, chlorine and/or methyl,

m represents the numbers 0, 1, 2 or 3,

X represents nitrogen or a CH group and

Y represents oxygen, sulphur, SO or $SO_2$,

and their acid addition salts and metal salt complexes.

2. Process for the preparation of hydroxyethyl-azolyl derivatives of the formula (I) according to Claim 1 and their acid addition salts and metal salt complexes, characterized in that

a) oxiranes of the formula

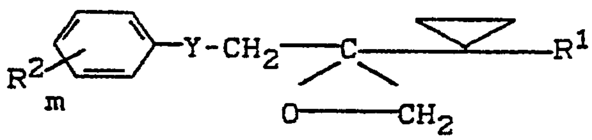

(II)

in which

$R^1$, $R^2$, Y and m have the abovementioned meaning, are reacted with azoles of the formula

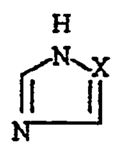

(III)

in which

X has the abovementioned meaning, in the presence of an acid-binding agent and in the presence of a diluent, or

b) azolyl derivatives of the formula

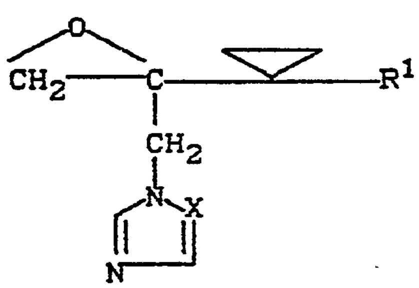

(IV)

in which

$R^1$ and X have the abovementioned meaning, are reacted with phenyl derivatives of the formula

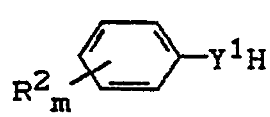

(V)

in which

$R^2$ and m have the abovementioned meaning and
$Y^1$ represents oxygen or sulphur, in the presence of an acid-binding agent and in the presence of a diluent, or
c) hydroxyethyl-azolyl derivatives of the formula

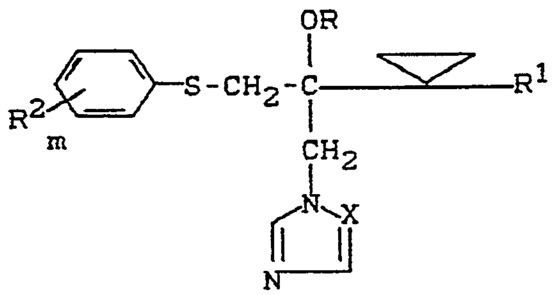

(Ia)

in which
R, $R^1$, $R^2$, X and m have the abovementioned meaning,
are reacted with oxidants, if appropriate in the presence of a diluent, or
d) hydroxyethyl-azolyl derivatives of the formula

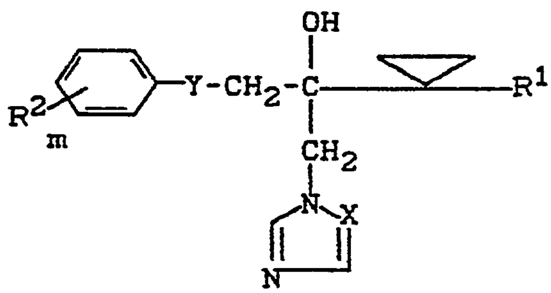

(Ib)

in which
$R^1$, $R^2$, X, Y and m have the abovementioned meaning, are reacted with strong bases in the presence of a diluent and the alcoholates of the formula

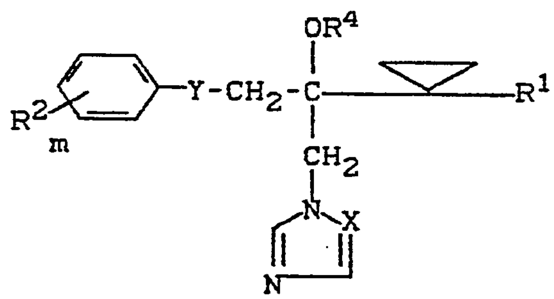

(Ic)

in which
$R^1$, $R^2$, X, Y and m have the abovementioned meaning, and
$R^4$ represents an alkali metal cation, or represents a quaternary ammonium or phosphonium cation, produced in this reaction are reacted with halogen compounds of the formula

$R^5$–Hal

in which
$R^5$ represents alkyl having 1 to 6 carbon atoms or alkylcarbonyl having 1 to 6 carbon atoms in the alkyl group and Hal represents chlorine or bromine, in the presence of a diluent,
and subsequently, if desired, an acid or a metal salt is added to the compounds of the formula (I) thus obtained.

3. Fungicides and plant growth regulating agents, characterized in that they contain at least one hydroxyethyl-azolyl derivative of the formula (I) according to Claim 1 or an acid addition salt or metal salt complex of a hydroxyethyl-azolyl derivative of the formula (I).

4. Use of hydroxyethyl-azolyl derivatives of the formula (I) according to Claim 1 or of their acid addition salts and metal salt complexes for combating fungi and for regulating plant growth.

5. Oxiranes of the formula

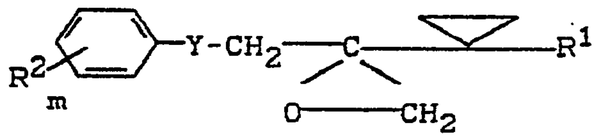

(II)

in which

$R^1$ represents fluorine, chlorine or bromine, or represents phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or represents the grouping $-Z-R^3$,

wherein

Z represents oxygen sulphur, SO or $SO_2$ and

$R^3$ represents phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms,

$R^2$ represents fluorine, chlorine, bromine, methyl, ethyl, isopropyl, tert.-butyl, methoxy, methylthio, trifluoromethyl, trifluoromethoxy, trifluoromethylthio, methoximinomethyl, phenyl which is optionally substituted by fluorine, chlorine and/or methyl, or represents phenoxy which is optionally substituted by fluorine, chlorine and/or methyl,

m represents the numbers 0, 1, 2 or 3, and

Y represents oxygen, sulphur, SO or $SO_2$.

6. Process for the preparation of oxiranes of the formula (II) according to Claim 5, characterized in that

e) cyclopropyl ketones of the formula

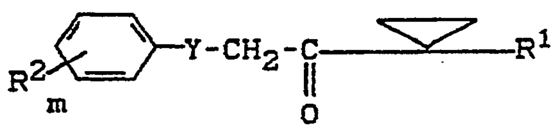

(VII)

in which

$R^1$, $R^2$, Y and m have the abovementioned meaning, are reacted either

($\alpha$) with dimethyloxosulphonium methylide of the formula

$$(CH_3)_2\overset{\delta\oplus}{S}O\overset{\delta\ominus}{C}H_2 \qquad (VIII)$$

or

$\beta$) with dimethylsulphonium methylide of the formula

$$(CH_3)_2\overset{\delta\oplus}{S}\quad\overset{\delta\ominus}{CH_2} \qquad (IX)$$

in the presence of a diluent.

7. Cyclopropyl ketones of the formula

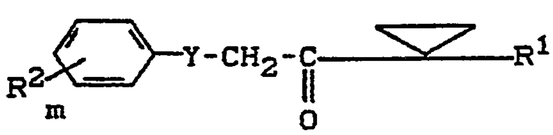

(VII)

in which

$R^1$ represents fluorine, chlorine or bromine, or represents phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or represents the grouping $-Z-R^3$,

wherein
Z represents oxygen, sulphur, SO or $SO_2$ and
$R^3$ represents phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms,
$R^2$ represents fluorine, chlorine, bromine, methyl, ethyl, isopropyl, tert.-butyl, methoxy, methylthio, trifluoromethyl, trifluoromethoxy, trifluoromethylthio, methoximinomethyl, phenyl which is optionally substituted by fluorine, chlorine and/or methyl, or phenoxy which is optionally substituted by fluorine, chlorine and/or methyl,
m represents the numbers 0, 1, 2 or 3, and
Y represents oxygen, sulphur, SO or $SO_2$.

8. Process for the preparation of cylcopropylketones of the formula (VII) according to Claim 7, characterized in that
f) halogenoketones of the formula

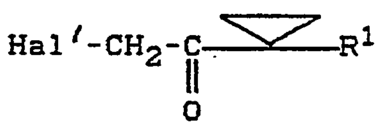

(X)

in which
$R^1$ has the abovementioned meaning and Hal' represents chlorine or bromine,
are reacted with phenyl derivatives of the formula

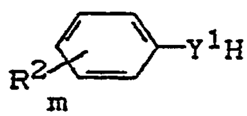

(V)

in which
$R^2$ and m have the abovementioned meaning, and
$Y^1$ represents oxygen or sulphur,
in the presence of an acid-binding agent and if appropriate in the presence of a diluent, and if appropriate the product is then oxidized.

9. Azolyl derivatives of the formula

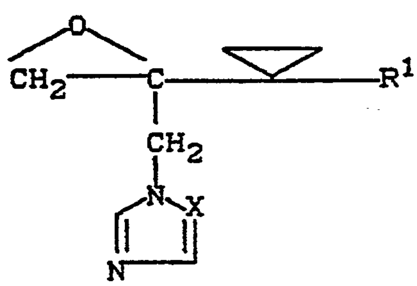

(IV)

in which
$R^1$ represents fluorine, chlorine, bromine, or phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or represents the grouping $-Z-R^3$, wherein
Z represents oxygen, sulphur, SO or $SO_2$,
$R^3$ represents phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, and
X represents nitrogen or a CH group.

10. Process for the preparation of azolyl derivatives of the formula (IV) according to Claim 9, characterized in that
h) azolylketones of the formula

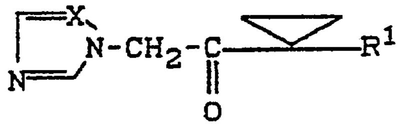

(XII)

in which
$R^1$ and X have the abovementioned meaning, are reacted either
($\alpha$) with dimethyloxosulphonium methylide of the formula

$$\overset{\delta\ominus}{(CH_3)_2SO}\ \overset{\delta\ominus}{CH_2} \qquad \text{(VIII)}$$

or
$\beta$) with dimethylsulphonium methylide of the formula

$$\overset{\delta\ominus}{(CH_3)_2S}\ \overset{\delta\ominus}{CH_2} \qquad \text{(IX)}$$

in the presence of a diluent.

11. Azolylketones of the formula

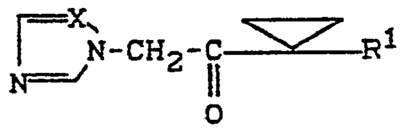

(XII)

in which
$R^1$ represents fluorine, chlorine, bromine, or represents phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, or represents the grouping $-Z-R^3$,
wherein Z represents oxygen, sulphur, SO or $SO_2$,
$R^3$ represents phenyl which is optionally substituted by halogen, halogenoalkyl having 1 or 2 carbon atoms and 1 to 5 halogen atoms, alkyl having 1 to 4 carbon atoms and/or alkoxy having 1 to 4 carbon atoms, and
X represents nitrogen or a CH group.

12. Process for the preparation of azolylketones of the formula (XII) according to Claim 11, characterized in that
i) halogenoketones of the formula

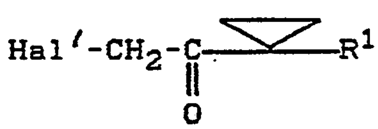

(X)

in which
$R^1$ has the abovementioned meaning and
Hal' represents chlorine or bromine,
are reacted with azoles of the formula

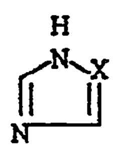

(III)

in which
X has the abovementioned meaning,

in the presence of an acid-binding agent and in the presence of a diluent.

**Revendications**

1. Dérivés hydroxyéthylazolyliques de formule

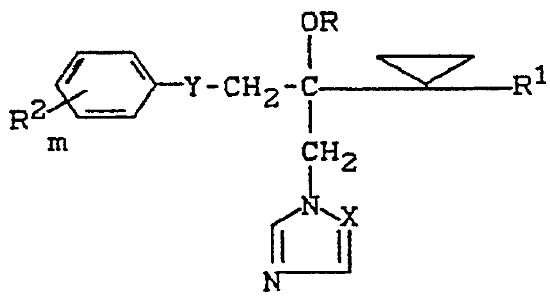

(I)

dans laquelle
R représente l'hydrogène, un groupe alkyle ayant 1 à 6 atomes de carbone ou un groupe alkylcarbonyle ayant 1 à 6 atomes de carbone dans le groupe alkyle,
$R^1$ représente le fluor, le chlore, le brome, un groupe phényle éventuellement substitué par un halogène, un radical halogénalkyle ayant 1 ou 2 atomes de carbone et 1 à 5 atomes d'halogènes, un radical alkyle ayant 1 à 4 atomes de carbone et/ou un radical alkoxy ayant 1 à 4 atomes de carbone, ou représente le groupement $-Z-R^3$, dans lequel
Z est l'oxygène, le soufre, un groupe SO ou $SO_2$ et
$R^3$ est un groupe phényle substitué le cas échéant par un halogène, un radical halogénalkyle ayant 1 ou 2 atomes de carbone et 1 à 5 atomes d'halogènes, un radical alkyle ayant 1 à 4 atomes de carbone et/ou un radical alkoxy ayant 1 à 4 atomes de carbone,
$R^2$ représente le fluor, le chlore, le brome, un groupe méthyle, éthyle, isopropyle, tertio-butyle, méthoxy, méthylthio, trifluorométhyle, trifluorométhoxy, trifluorométhylthio, méthoximinométhyle, un groupe phényle éventuellement substitué par du fluor, du chlore et/ou un radical méthyle ou un groupe phénoxy éventuellement substitué par du fluor, du chlore et/ou un radical méthyle,
m représente les nombres 0, 1, 2 ou 3,
X représente l'azote ou un groupe CH et
Y représente l'oxygène, le soufre, un groupe SO ou $SO_2$,
ainsi que leurs sels d'addition d'acides et leurs complexes de sels métalliques.

2. Procédé de production de dérivés hydroxyéthylazolyliques de formule (I) suivant la revendication 1, ainsi que de leurs sels d'addition d'acides et de leurs complexes de sels métalliques, caractérisé en ce que:
a) On fait réagir des oxirannes de formule

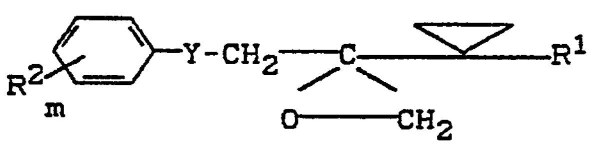

(II)

dans laquelle
$R^1$, $R^2$, Y et m ont la définition indiquée ci-dessus,
avec des azoles de formule

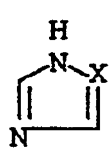

(III)

dans laquelle

X a la définition indiquée ci-dessus, en présence d'un accepteur d'acide et en présence d'un diluant, ou bien

b) On fait réagir des dérivés azolyliques de formule

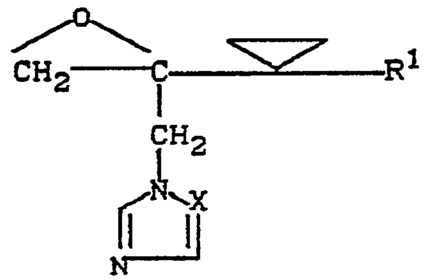

(IV)

dans laquelle

$R^1$ et X ont la définition indiquée ci-dessus,

avec des dérivés phényliques de formule

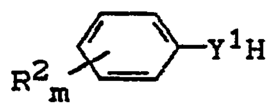

(V)

dans laquelle

$R^2$ et m ont la définition indiquée ci-dessus et

$Y^1$ représente l'oxygène ou le soufre,

en présence d'un accepteur d'acide et en présence d'un diluant, ou bien

c) On fait réagir des dérivés hydroxyéthylazolyliques de formule

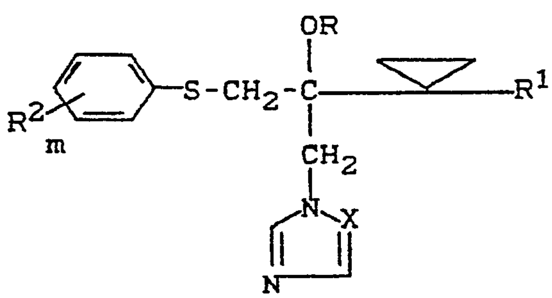

(Ia)

dans laquelle

R, $R^1$, $R^2$, X et m ont la définition indiquée ci-dessus,

avec des oxydants, le cas échéant en présence d'un diluant, ou bien

d) On fait réagir des dérivés hydroxyéthylazolyliques de formule

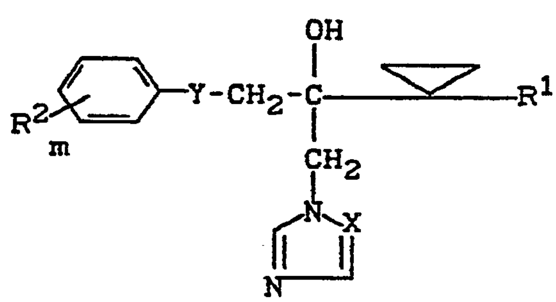

(Ib)

dans laquelle

$R^1$, $R^2$, X, Y et m ont la définition indiquée ci-dessus,

avec des bases fortes en présence d'un diluant et on fait réagir les alcoolates ainsi obtenus de formule

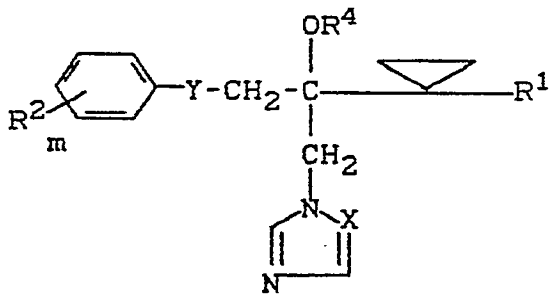

(Ic)

dans laquelle
$R^1$, $R^2$, X, Y et m ont la définition indiquée ci-dessus et
$R^4$ est un cation de métal alcalin ou un cation d'ammonium ou de phosphonium quaternaire, avec des composés halogénés de formule
$R^5$–Hal (VI)
dans laquelle
$R^5$ est un groupe alkyle ayant 1 à 6 atomes de carbone ou un groupe alkylcarbonyle ayant 1 a 6 atomes de carbone dans le groupe alkyle et
Hal représente le chlore ou le brome, en présence d'un diluant,
et le cas échéant, on additionne ensuite sur les composés de formule (I) ainsi obtenus un acide ou un sel métallique.

3. Compositions fongicides et régulatrices de croissance des plantes, caractérisées par une teneur en au moins un dérivé hydroxyethylazolylique de formule (I) suivant la revendication 1 ou en un sel d'addition d'acide ou un complexe de sel métallique d'un dérivé hydroxyéthylazolylique de formule (I).

4. Utilisation de dérivés hydroxyéthylazolyliques de formule (I) suivant la revendication 1 ou de leurs sels d'addition d'acides et de leurs complexes de sels métalliques pour combattre des champignons ainsi que pour influencer la croissance de plantes.

5. Oxirannes de formule

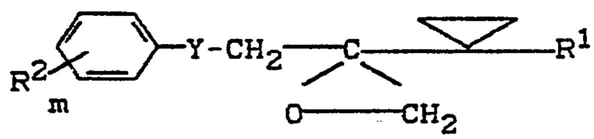

(II)

dans laquelle
$R^1$ représente le fluor, le chlore, le brome, un groupe phényle éventuellement substitué par un halogène, un radical halogénalkyle ayant 1 ou 2 atomes de carbone et 1 à 5 atomes d'halogènes, un radical alkyle ayant 1 à 4 atomes de carbone et/ou un radical alkoxy ayant 1 à 4 atomes de carbone, ou le groupement $-Z-R^3$, dans lequel
Z est l'oxygene, le soufre, un groupe SO ou $SO_2$ et
$R^3$ est un groupe phényle éventuellement substitué par un halogène, un radical halogénalkyle ayant 1 ou 2 atomes de carbone et 1 à 5 atomes d'halogenes, un radical alkyle ayant 1 à 4 atomes de carbone et/ou un radical alkoxy ayant 1 à 4 atomes de carbone,
$R^2$ est le fluor, le chlore, le brome, un radical méthyle, éthyle, isopropyle, tertio-butyle, méthoxy, méthylthio, trifluorométhyle, trifluorométhoxy, trifluorométhylthio, méthoximinométhyle, phényle portant éventuellement un substituant fluoro, chloro et/ou méthyle, ou phénoxy portant éventuellement un substituant fluoro, chloro et/ou méthyle.
m représente les nombres 0, 1, 2 ou 3 et
Y est l'oxygène, le soufre, un groupe SO ou $SO_2$.

6. Procédé de production d'oxirannes de formule (II) suivant la revendication 5, caractérisé en ce que
e) On fait réagir des cyclopropylcétones de formule

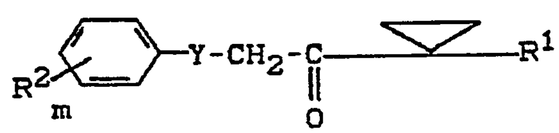

(VII)

dans laquelle
$R^1$, $R^2$, Y et m ont la définition indiquée

ci-dessus,
ou bien
α) avec le méthylure de diméthyloxosulfonium de formule

$$\overset{\delta\ominus\ \delta\ominus}{(CH_3)_2SOCH_2} \qquad (VIII)$$

ou bien
β) avec le méthylure de diméthylsulfonium de formule

$$(CH_3)_2\overset{\delta\ominus}{S}\ \ \overset{\delta\ominus}{CH_2} \qquad (IX)$$

en présence d'un diluant.

7. Cyclopropylcétones de formule

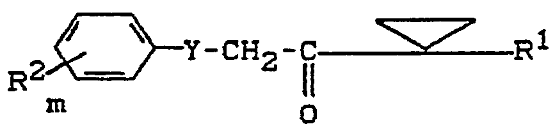

(VII)

dans laquelle
$R^1$ représente le fluor, le chlore, le brome, un groupe phényle éventuellement substitué par un halogène, un radical halogénalkyle ayant 1 ou 2 atomes de carbone et 1 a 5 atomes d'halogènes, un radical alkyle ayant 1 à 4 atomes de carbone et/ou un radical alkoxy ayant 1 a 4 atomes de carbone, ou le groupement –Z–$R^3$, dans lequel
Z représente l'oxygène, le soufre, un groupe SO ou $SO_2$ et
$R^3$ est un groupe phényle éventuellement substitué par un halogène, un radical halogénalkyle ayant 1 ou 2 atomes de carbone et 1 à 5 atomes d'halogènes, un radical alkyle ayant 0 1 à 4 atomes de carbone et/ou un radical alkoxy ayant 1 à 4 atomes de carbone,
$R^2$ représente le fluor, le chlore, le brome, un radical méthyle, éthyle, isopropyle, tertio-butyle, méthoxy, méthylthio, trifluorométhyle, trifluorométhoxy, trifluorométhylthio, méthoxyminométhyle, phényle portant éventuellement un substituant fluoro, chloro et/ou méthyle, ou phénoxy portant éventuellement un substituant fluoro, chloro et/ou méthyle,
m représente les nombres 0, 1, 2 ou 3 et
Y représente l'oxygène, le soufre, un groupe SO ou $SO_2$.

8. Procédé de production de cyclopropylcétones de formule (VII) suivant la revendication 7, caractérisé en ce que
f) On fait réagir des halogénocétones de formule

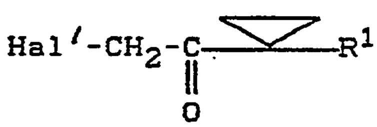

(X)

dans laquelle
$R^1$ a la définition indiquée ci-dessus et
Hal' représente le chlore ou le brome, avec des dérivés phényliques de formule

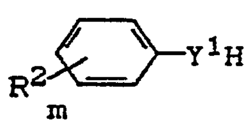

(V)

dans laquelle
$R^2$ et m ont la définition indiquée ci-dessus et
$Y^1$ est l'oxygène ou le soufre, en présence d'un accepteur d'acide et le cas échéant en présence d'un diluant, et on effectue ensuite éventuellement une oxydation.

9. Dérivés azolyliques de formule

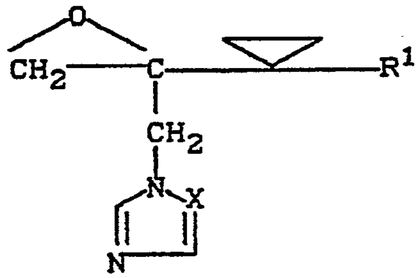

(IV)

dans laquelle
$R^1$ représente le fluor, le chlore, le brome, un groupe phényle éventuellement substitué par un halogène, un radical halogénalkyle ayant 1 ou 2 atomes de carbone et 1 a 5 atomes d'halogènes, un radical alkyle ayant 1 à 4 atomes de carbone et/ou un radical alkoxy ayant 1 à 4 atomes de carbone, ou le groupement –Z–$R^3$, dans lequel Z représente l'oxygène, le soufre, un groupe SO ou $SO_2$ et
$R^3$ est un groupe phényle éventuellement substitué par un halogène, un radical halogénalkyle ayant 1 ou 2 atomes de carbone et 1 à 5 atomes d'halogènes, un radical alkyle ayant 1 à 4 atomes de carbone et/ou un radical alkoxy ayant 1 à 4 atomes de carbone, et
X est l'azote ou un groupe CH.

10. Procédé de production de dérivés azolyliques de formule (IV) suivant la revendication 9, caractérisé en ce que
h) On fait réagir des azolylcétones de formule

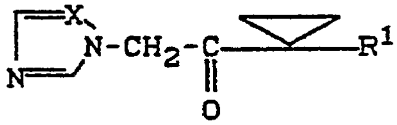

(XII)

dans laquelle
$R^1$ et X ont la définition indiquée ci-dessus,
ou bien
α) avec le méthylure de diméthyloxosulfonium de formule

$$\overset{\delta^{\ominus}}{(CH_3)_2SO}\ \overset{\delta^{\ominus}}{CH_2} \qquad (VIII)$$

ou bien
β) le méthylure de diméthylsulfonium de formule

$$\overset{\delta^{\ominus}}{(CH_3)_2S}\ \overset{\delta^{\ominus}}{CH_2} \qquad (IX)$$

en présence d'un diluant.

11. Azolylcétones de formule

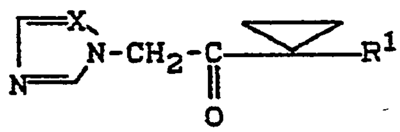

(XII)

dans laquelle
$R^1$ représente le fluor, le chlore, le brome, un groupe phényle éventuellement substitué par un halogène, un radical halogénalkyle ayant 1 ou 2 atomes de carbone et 1 à 5 atomes d'halogènes, un radical alkyle ayant 1 à 4 atomes de carbone et/ou un radical alkoxy ayant 1 à 4 atomes de carbone, ou le groupement –Z–$R^3$, dans lequel

Z représente l'oxygène, le soufre, un groupe SO ou $SO_2$ et
$R^3$ est un groupe phényle éventuellement substitué par un halogène, un radical halogénalkyle ayant 1 ou 2 atomes de carbone et 1 à 5 atomes d'halogènes, un radical alkyle ayant 1 à 4 atomes de carbone et/ou un radical alkoxy ayant 1 à 4 atomes de carbone, et
X est l'azote ou un groupe CH.

12. Procédé de production d'azolylcétones de formule (XII) suivant la revendication 11, caractérisé en ce que
i) On fait réagir des halogénocétones de formule

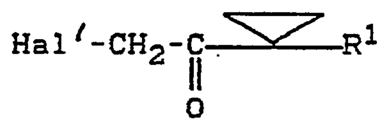

(X)

dans laquelle
$R^1$ a la définition indiquée ci-dessus et
Hal' représente le chlore ou le brome, avec des azoles de formule

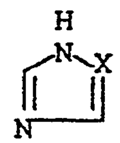

(III)

dans laquelle
X a la définition indiquée ci-dessus, en présence d'un accepteur d'acide et en présence d'un diluant.